# EUROPÄISCHE PATENTSCHRIFT

(11) **EP 3 980 780 B1**
(45) Veröffentlichungstag und Bekanntmachung des Hinweises auf die Patenterteilung: **23.08.2023**
(21) Anmeldenummer: 20757519.2
(22) Anmeldetag: 06.07.2020
(51) Int. Cl.: G01N 33/543, G01N 21/00, G01N 33/558, G01N 21/84

(54) **VORRICHTUNG ZUR AUSLESUNG EINES TEST-KITS ZUM NACHWEIS VON BIOMARKERN**
DEVICE FOR READING OUT A TEST KIT FOR DETECTING BIOMARKERS
DISPOSITIF POUR SÉLECTIONNER UN KIT DE TEST POUR METTRE EN ÉVIDENCE LA PRÉSENCE DE BIOMARQUEURS

(30) Priorität: 05.07.2019 DE 102019004630
(43) Veröffentlichungstag der Anmeldung: 13.04.2022
(73) Patentinhaber: ScheBo Biotech AG, 35394 Giessen (DE)
(72) Erfinder: SCHEEFERS, Hans, 35438 Wettenberg (DE)
(74) Vertreter: Jungblut & Seuss
(86) Internationale Anmeldenummer: PCT/DE2020/000150
(87) Internationale Veröffentlichungsnummer: WO 2021/004564

(56) Entgegenhaltungen:
- EP-A1- 2 587 249
- WO-A1-2018/150787
- US-A1- 2010 267 049

## Beschreibung

Die vorliegende Patentanmeldung nimmt die Priorität der deutschen Anmeldung DE 102019 004 630.7 (Anmeldetag:5.7.2019) in Anspruch.

### Gebiet der Erfindung

Die Erfindung betrifft eine gewerblich anwendbare Vorrichtung zur Auslesung eines Test-Kits zum Nachweis von Biomarkern, insbesondere zum Nachweis von Biomarkern im menschlichen und/ oder tierischen Stuhl. Die Vorrichtung ist dazu geeignet und bestimmt, den qualitativen und/oder quantitativen Nachweis der Biomarker zu objektivieren. Die Erfindung betrifft im Besonderen eine Vorrichtung, in die ein Teststreifen eines immunchromatografischen Nachweisverfahrens ortsfest eingelegt wird. Der Teststreifen wird durch näher definierte Beleuchtungseinrichtungen beleuchtet und das von der Oberfläche des Teststreifens reflektierte Licht durch einen näher spezifizierten Sensor aufgezeichnet. Mittels eines speziellen Beleuchtungs- und Ausleseverfahrens durch digitale Bildverarbeitung erfolgt eine präzise Auslesung des angezeigten Testergebnisses.

### Hintergrund der Erfindung und Stand der Technik

Es ist bekannt, zur Darmkrebsvorsorge ein Screening von Probanden durchzuführen. Ein derartiges Screening kann beispielsweise mit einen immunchromatografischen Schnelltest durchgeführt werden. Im Rahmen eines solchen Tests wird eine aufbereitete Stuhlprobe auf einen immunchromatografischen Teststreifen (Lateral-Flow Verfahren) aufgetragen. Mittels eines auf dem Teststreifen fixierten Antikörpers können bestimmte als Biomarker dienende Antigene (z.B. Tumor M2-PK) durch eine Agglutinationsreaktion sichtbar gemacht werden. Hierbei kommt es zu einer lokalen Ansammlung von Partikel (z.B. Goldpartikeln), die mit entsprechenden Antikörpern beschichtet sind. Eine genauere Beschreibung inklusive weiterer Verweise findet sich in WO 2014/008884 A1. Die beschriebene Agglutinationsreaktion führt zu einer dunklen, üblicherweise linienförmigen, Markierung auf dem üblicherweise hellen Teststreifen, welcher visuell detektiert wird. Hierbei tritt das Problem auf, dass die subjektive Beurteilung der erhaltenen Linie Schwankungen unterworfen ist. Die Wahrnehmung hängt sowohl vom individuellen Sehvermögen des Ablesers ab, als auch von Helligkeit und Art des Lichteinfalls. So kann bei Kunstlicht dieselbe Linie anders wahrgenommen werden als bei Sonnenlicht. Auch die verschiedenen Kunstlichtarten (Glühlampe, Halogenlampe, Leuchtstoffröhre, LED, etc), bedingt durch unterschiedliche spektrale Lichtverteilung, können je nach Lichtquelle anders wahrgenommen werden. Die Wahrnehmung von verschiedenen Farben ist darüber hinaus sehr subjektiv. Besonders problematisch wird das Erkennen der Testlinie bei Proben, deren Biomarkerkonzentration im Bereich des Cut-offs liegen.

Der Begriff Cut-off bzw. Toleranzgrenze bezeichnet dabei einen Toleranzwert in der Analytik, z.B. von Biomarkern. Er legt fest, ab wann ein Testergebnis positiv bzw. negativ zu beurteilen ist. Entsprechende Probleme treten auch in anderen Testsystemen auf.

Um die erhaltenen Messergebnisse zu objektivieren, sind bereits Lesegeräte beschrieben worden. Diese Lesegeräte (Reader) nutzen unterschiedliche physikalische Messmethoden. Es wurden auch bereits Lesegeräte beschrieben, die das Kamerasystem handelsüblicher Smartphones verwenden. Die beschriebenen Lesegeräte sind jedoch unkalibriert und/oder nicht auf den speziellen Anwendungsbereich angepasst, so dass die Qualität der Messungen, insbesondere die Reproduzierbarkeit, unzureichend ist. Wünschenswert wäre daher eine Vorrichtung, die in vollautomatischer Weise ein Messergebnis in reproduzierbarer Form liefert und somit dem Fachpersonal, insbesondere dem Arzt, das Messergebnis objektiv aufbereitet. Die Untersuchung/das Verfahren soll zeit- und kostengünstig sein. Die Untersuchung soll den zu Untersuchenden möglichst wenig belasten. Die mit der Vorrichtung durchgeführte Untersuchung/Messung dient der Steigerung der Qualitätssicherung.

Nach dem Stand der Technik werden seit Jahren Teststreifen (Lateral Flow-Teststreifen) und die dazugehörigen Testverfahren zur Quantifizierung von z.B. Biomarkern in einer Probandenprobe (Blut, Plasma, Urin, Stuhl, Sperma, sonstige Körperflüssigkeiten, wässriger Extrakt) verwendet. Ähnliche Tests sind bekannt für Zwecke der Umweltanalytik, der Nahrungsmittelanalytik, der forensischen Analytik, inklusive Dopingtests, Drogen(schnell)tests oder für Schwangerschaftstests.

Die Beurteilung des Ergebnisses bei diesem Teststreifen und Verfahren zur Beurteilung und zur Interpretation des Testergebnisses basiert auf der Wahrnehmung des Vorhandenseins oder Nicht-Vorhandenseins einer subjektiv sichtbaren Linie im Test-Mess-Bereich, im Erwartungsbereich (T), im Vergleich zu einem Referenz-Bereich auf dem Messstreifen durch in Augenscheinnahme unter Verwendung des verfügbaren Lichtes (ambient light). Die Beurteilung, Interpretation (positives Ergebnis oder negatives Ergebnis), d.h. Linie erkennbar (vorhanden) oder nicht erkennbar (nicht vorhanden) negatives Testergebnis.

Die Beurteilung, Interpretation des Testergebnisses auf dem Teststreifen bezieht sich auf die subjektive Sinneswahrnehmung einer Person, die sich die Funktionsschicht, den Messstreifen, den Teststreifen, ansieht. Physiologisch erfolgt die Beurteilung des Teststreifens durch Abgleich "vorhandener Daten" im Gehirn, d. h. durch sensorische Integration. Die Beurteilung, Interpretation wird nicht die Lichtfarbe sondern die Körperfarbe, d.h. das vom beleuchteten Körper (Funktionsschicht, Teststreifen) reflektierte Licht.

Die Interpretation erfolgt durch die Testperson aufgrund eines Farbreizes. Der Farbreiz beruht auf der spektralen Zusammensetzung des reflektierten Lichtes der Körperfarbe. Die Körperfarbe ist ein Lichtgemisch mehrerer Wellenlängen/Bereichen und Intensitätsverteilungen. In Verbindung mit den Fotorezeptoren im menschlichen Auge und der Verarbeitung im Nervensystem hat jede Körperfarbe eine bestimmte Charakteristik, die Farbvalenz eine physiologische Kenngröße.

Die "physiologische Messung" der Farbvalenz orientiert sich an den physiologischen Wahrnehmungseigenschaften des menschlichen Auges, d.h. insbesondere der Testperson, die den Messstreifen auslesen soll. Welche Farbe, Intensität von der Person empfunden wird, hängt von der Spektralverteilung und Intensität des reflektierten Lichtes, des Teststreifens, der Körperfarbe des Teststreifens ab. Die Farbwahrnehmung und deren Intensität ist ein komplexer physiologischer personenbezogener individueller Prozess, bei dem zahlreiche Nicht-Linearitäten sowie Kontrast- und Nachwirkungseffekte einfließen, welche sich nicht kalibrieren und objektiv nicht erfassen und dokumentieren lassen.

Zudem kommt, dass die Farbe der Teststreifen (Körperfarbe) unterschiedlich bei unterschiedlichen Proben von denselben Probanden sich darstellt.

Diese Eigenschaften und die unterschiedliche subjektive Wahrnehmung der Person, die den Schnelltest/den Teststreifen in Augenschein nimmt können zur falschen Beurteilung/Interpretation und damit zu ungesicherten Sensitivität/Spezifität und damit zur ungesicherten Treffsicherheit bezüglich der Aussage des Testsystems führen.

Die technische Aufgabe besteht darin, ein System/eine Apparatur/eine Vorrichtung bereitzustellen, die es erlaubt, standardisiert, objektiviert, die automatisiert die Verfärbung in der Funktionsschicht/Teststreifen zu erfassen.

Darüber hinaus erfolgt die unterschiedliche Verfärbung in der Funktionsschicht zeitabhängig. Dieses sei an einem Beispiel erläutert:
Nach Auftragen des wässrigen Extraktes der Probe auf die Auftragsstelle des Teststreifens bewegt sich die Flüssigkeitsfront in Richtung des Wicks. Dabei beobachtet man, dass sich das kolloidale Gold sichtbar an der Rosafärbung des Teststreifens Richtung Wick bewegt. Im Falle einer "positiven Probe" ist innerhalb der Erwartungsregion eine gleichmäßige Rosafärbung zu sehen. Mit der Zeit verschwindet die gleichmäßige Rosafärbung des Teststreifens in der Erwartungsregion und langsam wird eine Linie in der Testregion/in der Erwartungsregion über die Zeit sichtbar. Die Linie ist sehr gut sichtbar vor dem Hintergrund.

Bei einer negativen Probe entsteht in der Erwartungsregion/ Testregion keine Linie. Ist die Konzentration des zu bestimmenden Biomarkers sehr niedrig, d.h. die Immunaggregation in der Funktionsschicht/in der Erwartungsregion erfolgt nur geringfügig.

Diese Situation mit falsch-positiven bzw. falsch-negativen Ergebnissen ist besonders häufig bei Proben mit Biomarker-Konzentrationen, die sich nahe am cut-off befinden. Des Weiteren kann z.B. auch eine sehr schwach auftretende Linie nach einer gewissen Zeit - länger als z.B. 10 Minuten - wieder verschwinden, da der Untergrund wieder rosa wird und die Linie im Untergrund verschwindet. Ein Grund hierfür ist die Umkehr der Laufrichtung. Während zu Anfang nach Auftragen der Probe durch Kapillarwirkung bedingt die Flüssigkeitsfront Richtung Wick läuft, findet nachher eine Umkehr der Bewegungsrichtung statt, da das Wick nicht mehr genügend Kapillarwirkung auf die Funktionsschicht ausübt, und nun die Kapillarkräfte der Auftragsregion stärker werden als die Kapillarwirkung des Wicks.

Zur optimalen Beurteilung des Teststreifens ist es daher besonders wichtig, immer eine konstante Zeit nach dem Auftragen der Probe einzuhalten.

Die technischen Aufgaben bestehen darin, die o.g. Mängel durch der Bereitstellung einer erfindungsgemäßen Vorrichtung zu beseitigen.

Die Patentanmedlung US2010/267049 enthüllt eine Vorrichtung zur Auslesung eines Test-Kits zum Nachweis von Biomarkern, aber nicht das Merkmal, dass "die Signalaufbereitung nur das Helligkeitsignal (Y-Signal) des Empfängers auswertet".

WO2018/150787 enthüllt eine Vorrichtung zur Auslesung eines Test-Kits zum Nachweis von Biomarkern aber nicht die Verwendung von mindestens 2 verschiedenen Wellenlängenbereichen.

EP2587249 enthüllt eine Vorrichtung zum Auslesen eines Test-Kits, wobei zwei Lichtquellen verwendet werden und die Lichtstärke der Reflexion gemessen wird aber die verwendeten Wellenlängen der ersten und zweiten Lichtquellen sind identisch.

### Grundzüge der Erfindung und bevorzugte Ausführungsformen

Es wurde gefunden, dass eine Vorrichtung zur Auslesung eines Test-Kits zum Nachweis von Biomarkern, enthaltend
- ein lichtundurchlässiges Gehäuse,
- eine Haltevorrichtung zum Fixieren einer Funktionsschicht mit oder ohne Kassette
- eine Funktionsschicht mit darauf fixierten Antikörpern zum immunologischen Nachweis eines Antigens und/oder eine Funktionsschicht mit darauf fixierten Antigenen zum immunologischen Nachweis eines Antikörpers,
- einen Sender zur gesteuerten Beleuchtung der Funktionsschicht, wobei der Sender gesteuert Licht unterschiedlicher Wellenlänge aussenden kann,
   - einen Empfänger zur Bestimmung des reflektierten oder transmittierten Lichtes,
   - einen Prozessor zur Steuerung von Sender, Empfänger und zur Signalaufbereitung,
   - eine Anzeigevorrichtung zur Anzeige des Ergebnisses,
   wobei die Vorrichtung mittels einer auf dem Prozessor ablaufenden Software gesteuert wird,
   wobei die Steuerung
   - den Sender nacheinander Licht von mindestens 2 verschiedenen Wellenlängenbereichen aussenden lässt,
   - die Signalaufbereitung nur das Helligkeitsignal (Y-Signal) des Empfängers auswertet,
   - den Messzyklus ein- oder mehrfach (vorzugsweise 2 - 5-fach) wiederholt,
   - die gemessenen Signale per digitaler Bildverarbeitung auswertet,
   die eingangs geschilderten Probleme überwindet.

Die erfindungsgemäße Vorrichtung enthält daher:
- eine Funktionsschicht zum immunologischen Nachweis eines Antigens,
- einen Sender (Lichtquelle) zur gesteuerten Belichtung der Funktionsschicht,
- einen Empfänger zur Bestimmung des reflektierten Lichtes,
- einen Prozessor zur Steuerung von Sender, Empfänger und zur Signalaufbereitung,
- eine Anzeigevorrichtung zur Anzeige des Ergebnisses,
- ein Gehäuse zum Einlegen der Funktionsschicht und zum Ausschluss anderer Lichtquellen.

Weitere Ausgestaltungen der Erfindung werden nachfolgend beschrieben und sind teilweise Gegenstand von Unteransprüchen.

Der Sender (Lichtquelle) der erfindungsgemäßen Vorrichtung muss in der Lage sein, Licht verschiedener Wellenlängenbereiche zu emittieren. Möglich wäre es, eine Weißlichtquelle, z.B. eine herkömmliche Glühlampe, zu verwenden, die mit verschiedenfarbigen Filtern ausgestattet ist. Erfindungsgemäß bevorzugt werden hierzu LEDs mit verschiedenen Farben (z.B. Rot, Grün, Blau) verwendet. Bevorzugt werden LED-Module herangezogen (z.B. RGB-LED-Module), wie sie beispielsweise in LED-Anzeigevorrichtungen (Bildschirmen) verbaut sind. Die LEDs können auch in OLED-Technologie hergestellt sein. Derartige Module, wie sie z.B. in Smartphone-Displays realisiert sind, bilden üblicherweise ein Array von LEDs, deren Größe jeweils im Submillimeterbereich liegt und die einzeln ansteuerbar sind (Pixel). Mittels derartiger LED-Arrays können daher lokal bestimmte Lichtemissionen unterschiedlicher Wellenlängen erzeugt werden. Die Verwendung derartiger LED-Arrays erfolgt auch aus wirtschaftlichen Gründen, weil diese als Massenprodukt kostengünstig erhältlich sind. Im Gegensatz zu einer Verwendung als Display werden im Rahmen der erfindungsgemäßen Vorrichtung im Regelfall nur bestimmte Pixel zum Leuchten angeregt.

Geeignete Lichtquellen hierfür sind beispielsweise adressierbare RGB-LEDs, wie sie unter der Bezeichnung WS2812 (Neopixel) kommerziell angeboten werden. Das durch WS2812 abgestrahlte Licht hat sich als relativ schmalbandig herausgestellt, mit Peaks bei:

| | |
|---|---|
| rot: | 620-630 nm |
| grün: | 515-530 nm |
| blau: | 465-475 nm |

Eine besondere Variante dieser Module besteht in der Verwendung von sogenannten RWGB-Modulen (4-in-1-LED-Modul oder 5-in-1-Multicolor-Modul) .

Eine RWGB-Modul-Variante kann sowohl die drei Primärfarben Rot mit etwa 620 nm, Grün mit 565 nm und Blau mit 485 nm, kurzwelliges Blau als auch weißes Licht mit z.B. 3200 Kelvin emittieren. Eine JISD-Modul-Variante besteht aus mehreren LED-Segmenten, die von Warm- bis Kaltweiß mit den Farbtemperaturen z.B. 2700, 3000, 4000, 5700 Kelvin ausstrahlen.

Verwendet werden können auch Leuchtmittel, die im Nah-Infrarot-Bereich strahlen (700-900nm) und oder im UV-Bereich (400-315 nm).

Es besteht erfindungsgemäß die Möglichkeit, entsprechend der technischen Anforderung bezüglich Farbe und Farbtemperatur die Module individuell als auch während der Messung zur Verfügung zu stellen. Da erfindungsgemäß alle Module digital ansteuerbar sind, lässt sich während der Messzeit sowohl die Intensität als auch die Farbzusammensetzung dynamisch entsprechend den Messanforderungen während der Messvorgänge digital kontrolliert steuern.

Interessanterweise wurde festgestellt, dass es nicht nötig ist streng monochromatisches Licht (Licht mit einer einzigen definierten Wellenlänge) zu verwenden, wie es typischerweise von Lasern ausgestrahlt wird. Für den erfindungsgemäßen Zweck ist es ausreichend, wenn die einzelnen Lichtfarben Gemische verschiedenster Wellenlängen darstellen. Allerdings müssen die zwei oder mehr unterschiedlichen Lichtfarben voneinander verschieden sein.

Als Funktionsschicht dient insbesondere eine Lateral-Flow Membran, wie sie im Stand der Technik beschrieben ist. Alternativ können natürlich auch Western-Blot-Membranen oder ELISA-Wells herangezogen werden. Die immunchemische Bestimmung des jeweiligen Antigens erfolgt durch entsprechende antikörperbeschichtete Partikel, z.B. Goldpartikel. Alternativ kommen auch andere Partikel, wie z.B. Latexpartikel in Frage. Durch die bekannte Festphasenkopplung der Antikörper kommt es in dem Bereich, in dem die festphasengebundenen Antikörper gebunden sind im positiven Nachweisfall zu der beschriebenen Agglutinierungsreaktion, die aufgrund der Partikel zu einer wahrnehmbaren Verdunkelung führt. Die Funktionsschicht, z.B. in Form eines Lateral-Flow Teststreifens, kann dabei durchaus in eine Halterung eingebaut sein, wie z.B. eine handelsübliche Test-kassette. Derartige Testkassetten enthalten selbstverständlich angepasste Öffnungen zum Auftragen der Probenflüssigkeit und zum Ablesen des Testergebnisses (inkl. eines Kontrollfeldes). Die Funktionsschicht kann intransparent oder zumindest teilweise transparent sein. Als Träger kann beispielsweise eine Aluminiumfolie (intransparent) oder eine Polyacetatfolie (transparent) dienen. Im Fall einer intransparenten Funktionsschicht muss die erfindungsgemäße Messung durch Messung des reflektierten Lichtes erfolgen. Im Fall einer transparenten Funktionsschicht kann die erfindungsgemäße Messung auch durch Messung des transmittierten Lichtes erfolgen. Die erfindungsgemäße Vorrichtung kann natürlich auch mehrere Empfängermodule an unterschiedlichen Positionen aufweisen.

Geeignete Funktionsschichten hierfür sind beispielsweise die aus Lateral-Flow Testsystemen bekannten Nitrozelluloseschichten auf Aluminiumstreifen.

Weitere geeignete Funktionsschichten sind die aus der Dünnschichtchromatographie bekannten Trennschichten (stationäre Phase) bestehend aus einer dünnen Schicht eines sehr feinkörnigen Materials (z. B. Kieselgel, Kieselgur, Aluminiumoxid, Cellulose). Diese Trennschicht ist sehr gleichmäßig auf eine Trägerfolie oder Trägerplatte aus Kunststoff, Aluminiumblech oder Glas aufgetragen und kommerziell in unterschiedlichen Schichtdicken erhältlich. In der Regel kommt als stationäre Phase Kieselgel zum Einsatz (Normalphasenchromatographie), das aufgrund der freien endständigen Hydroxygruppen als polares Adsorbens für die Probenmoleküle dient. Der mittlere Porendurchmesser der Kieselgele beträgt meist 4 bis 100 nm, wobei der Porendurchmesser von 6 nm (Kieselgel 60, Merck) am gebräuchlichsten ist. Kieselgele enthalten Siloxan- oder Silanol-Gruppen.

Alternativ dazu kommen auch DC-Materialien mit anderen funktionellen Gruppen (z. B. Aminogruppen) zum Einsatz. Sie unterscheiden sich vom Standard-Kieselgel nicht nur in ihrer Polarität, sondern auch in der Basizität und führen damit zu völlig anderen Trennergebnissen. Auch oberflächenmodifizierte Kieselgele mit unpolaren Haftstellen (durch Kupplung mit Organochlorsilanen) werden eingesetzt (Umkehrphasenchromatographie, reversed phase). Die Reihenfolge, in der die verschiedenen Probemoleküle aufgetrennt werden, kehrt sich dann um - die polaren Moleküle laufen schneller, die unpolaren Moleküle werden stärker festgehalten. Vorteilhaft ist dabei unter anderem, dass auch sehr polare Proben untersucht werden können. Als weitere stationäre Phasen für die DC eignen sich auch Aluminiumoxid, Magnesiumsilikat, Kieselgur, Polyamid, Cellulose.

Weiterhin alternativ können auch poröse Glasschichten verwendet werden. Derartige poröse Glasschichten können ebenfalls in bestimmten Regionen mit Fängermolekülen versehen werden. Hierzu notwendige silanbasierte Linkertechnologien sind im Stand der Technik beschrieben. Ferner können auch FRET-basierte Immunoassays verwendet werden (mit oder ohne Quantum Dots).

Je nach Ausgestaltung der Funtionsschicht können unterschiedliche Biomarker detektiert werden. Typischerweise umfasst die Palette von möglichen Biomarkern Proteine, Lipide, Enzyme, Kohlenhydrate, Hormone, Stoffwechselprodukte, Medikamente und deren Abbauprodukte, aber auch Zellen oder Zellenbestandteile, Gene bzw. Gensequenzen und Viren.

Als Empfänger (Transducer oder Detektor) werden handelsübliche CCD- oder CMOS-Chips verwendet, wie sie in Digitalkameras verbaut sind. Derartige Chips sind ebenfalls als Array von einzelnen punktförmigen Lichtsensoren (Pixel) ausgebildet, die jeweils einzeln ausgelesen werden können. Die Verwendung derartiger CCD- bzw. CMOS-Chips erfolgt insbesondere aus wirtschaftlichen Gründen, weil diese als Massenprodukt kostengünstig erhältlich sind. Weiterentwicklungen dieser Chips, wie beispielsweise sCMOS Chips können selbstverständlich ebenfalls verwendet werden. Im Gegensatz zu einer Verwendung in einer Kamera werden im Rahmen der erfindungsgemäßen Vorrichtung jedoch nur einzelnen Pixelelemente ausgelesen (s. unten), so dass die Auslesung schnell und die Auswertung einfach ist. Darüber hinaus werden, wie weiter unten näher beschrieben, von den relevanten Pixeln bevorzugt nur die Helligkeitssignale (Y-Signale) ausgelesen. Die Beschränkung der weiteren Bildverarbeitung auf die relevanten Pixel und die Y-Signale führt zu einer erheblichen Vereinfachung und insbesondere Beschleunigung der digitalen Auswertung.

Im Fall einer intransparenten Funktionsschicht müssen Sender und Empfänger auf der gleichen Seite der Funktionsschicht angeordnet sein, beispielsweise oberhalb der Schicht.

Im Fall einer transparenten Funktionsschicht können Sender und Empfänger auf gegenüberliegenden Seiten der Funktionsschicht angeordnet sein, beispielsweise Sender oberhalb der Schicht, Empfänger unterhalb der Schicht.

Geeignete Empfänger hierfür sind insbesondere CMOS-Sensoren. Beim CMOS (Complementary Metal-Oxide Semiconductor)-Sensor werden die durch Photonen erzeugten Ladungen schon im Pixel in Spannung umgewandelt. Das ist der wesentliche Unterschied zum CCD-Sensor, bei dem die Ladungen erst aus dem Sensor zu einem Ausgangsverstärker verschoben werden müssen.

Bei den CMOS-Sensoren unterscheidet man zudem nach der Active- und Passive- Pixel-Technologie.

Bei der Active-Pixel-Technologie kann jeder Pixel getrennt angesteuert werden. Damit können alle Pixel zum gleichen Zeitpunkt belichtet werden (Global Shutter). Außerdem ist es hier - zumindest theoretisch - möglich, jeden Pixel getrennt auszulesen und so z.B. durch Wahl eines AOIs die Bildwiederholrate zu steigern.

Bei der Passive-Pixel-Technologie teilen sich die Pixel einer Zeile einen Teil der Elektronik. Die Belichtung und auch das Auslesen geschieht hier zeilenweise (Rolling Shutter).

Obwohl CMOS-Sensoren seit fast ebenso langer Zeit auf dem Markt erhältlich sind wie CCDs, hat die Leistungsfähigkeit erst vor kurzem ein Niveau erreicht, das den Einsatz in einem breiten Anwendungsbereich ermöglicht. Allerdings hat CMOS hinsichtlich Performance inzwischen CCD-Sensoren nicht nur eingeholt, sondern überholt.

Zu jeder einzelnen Fotodiode bzw. Pixel ist hier ein Kondensator parallel geschaltet, der durch den Fotostrom aufgeladen wird. Die erzeugte Spannung ist dabei proportional zur Helligkeit und zur Belichtungszeit.

Der grundlegende Unterschied zu den CCD-Sensoren ist, dass auf Schieberegister verzichtet wird und jedes Bildelement über eigene Ausleseverstärker bzw. Transistoren verfügt. Dies ermöglicht dem Anwender z.B. durch die Definition eines Teilbereichs des Sensors, einer so genannten "Region of Interest" (ROI), die Bildrate signifikant zu erhöhen.

Viele einfache CMOS-Sensoren mit Rolling-Shutter enthalten drei Transistoren (3T) pro Pixel; Zur Verbesserung der Funktionalität, z.B. zur Rauschunterdrückung, können weitere Transistoren (4T oder 5T) hinzugefügt werden. Dies geht jedoch zu Lasten des Füllfaktors und damit der Empfindlichkeit. Da jedes Pixel über eigene Verstärker verfügt, die aufgrund von Fertigungstoleranzen nicht einheitlich sind, entsteht eine als »Fixed Pattern Noise« bezeichnete Inhomogenität des gesamten Bildes, die durch eine Flat-Line- oder Flat-Field-Korrektur ausgeglichen werden kann.

CMOS-Sensoren lassen sich in zwei Unterkategorien aufteilen: Diejenigen mit A/D-Wandlung »On-Chip« und diejenigen ohne A/D-Wandlung »Off-Chip«. Das bedeutet, dass Sensoren mit A/D-Wandlung auf dem Sensor direkt digitale Signale ausgeben können, wodurch sich die gesamte Kameraelektronik reduzieren lässt und folglich Kosten eingespart werden können. Die Sensoren ohne A/D-Wandlungen hingegen geben analoge Signale aus, weshalb die Kameraelektronik mit einem zusätzlichen A/D-Wandler ausgelegt werden muss.

Das optisch auf den Bildsensor projizierte Bild der Funktionsschicht wird nach dem Stand der Technik mittels digitaler Signalverarbeitung und der diskreten Abtastwerte der einzelnen Pixel abgetastet. Die diskreten Abtastwerte der einzelnen Pixel werden nach Stand der Technik durch digitale Bildverarbeitungstechnik bearbeitet. Hierbei kommen gemäß Digitaltechnik unterschiedliche Filter (z.B. Box-Filter, Tiefpass-Filter, als auch unterschiedliche elektronische Bauelemente der Digitaltechnik, bspw. Logikgatter, Mikroprozessoren, Memristoren und Datenspeicher) zum Einsatz.

HDRC-Technologie im Bereich von CMOS-Bildsensoren (High-Dynamic-Ranch CMOS) kann nach Stand der Technik genutzt werden. Bei HDRC-Kameras wird für jeden Bildpunkt (Pixel) das lichtempfindliche Element (Photodiode) mit einem CMOS-Feldeffekttransistor kombiniert, um ein der Lichtintensität proportionales Signal zu erhalten.

Nach dem Stand der Technik erfolgt die Bildsensor-Kalibrierung in einem mehrstufigen Korrekturprozess des Rohbildes, des Sensors, mit Hilfe eines Weißbildes und eines Dunkelbildes.

Nach dem Stand der Technik wurde das Weißbild aufgenommen, während die Kamera auf eine gleichmäßig erhellte Fläche ausge Das optisch auf den Bildsensor projizierte Bild der Funktionsschicht wird nach dem Stand der Technik mittels digitaler Signalverarbeitung und der diskreten Abtastwerte der einzelnen Pixel abgetastet. Die diskreten Abtastwerte der einzelnen Pixel werden nach Stand der Technik durch digitale Bildverarbeitungstechnik bearbeitet. Hierbei kommen gemäß Digitaltechnik unterschiedliche Filter (z.B. Box-Filter, Tiefpass-Filter, als auch unterschiedliche elektronische Bauelemente der Digitaltechnik, bspw. Logikgatter, Mikroprozessoren, Memristoren und Datenspeicher) zum Einsatz.

HDRC-Technologie im Bereich von CMOS-Bildsensoren (High-Dynamic-Ranch CMOS) soll nach Stand der Technik genutzt werden. Bei HDRC-Kameras wird für jeden Bildpunkt (Pixel) das lichtempfindliche Element (Photodiode) mit einem CMOS-Feldeffekttransistor kombiniert, um ein der Lichtintensität proportionales Signal zu erhalten.

Das kalibrierte Bild kann zur Messung der scheinbaren Helligkeit herangezogen werden. Ohne Kalibrierung würde eine derartige Messung zu verfälschten Messwerten führen. Die scheinbare Helligkeit, wie sie dem menschlichen Auge mit visueller Wahrnehmung erscheint, wird als visuelle Helligkeit bezeichnet. Gemäß Fehlerkorrektur und zur Verhinderung von verschiedenen Effekten, z.B. durch Verschmutzung hervorgerufen, werden verschiedene Algorithmen zur Bildsensorkalibrierung nach dem Stand der Technik verwendet.

Beispielsweise kann das Kameramodul aus einem OV7670 mit eingebautem Signalprozessor bestehen, mit einer Auflösung von 640x480 pixel. Angesteuert wird das Modul durch den SCCB (serial camera control bus) vom Microcontroller aus. Andere Kameramodule mit gleichwertigen Eigenschaften sind ebenfalls einsetzbar. Für die Auswertung wird jeweils nur ein kleines Fenster (ca. 64x16 pixel) verwendet. Typischerweise wird ein derartiges Fenster für das Testfeld (die "Region of Interest" ROI) verwendet, ein weiteres Fenster für das Referenzfeld. Ebenso wird bevorzugt nur das Helligkeitssignal (Y-Wert des YUV-Signals) zur Auswertung herangezogen.

Zur Steuerung der Vorrichtung und Auswertung der Messung wird ein Mikroprozessor verwendet. Dabei ist beispielsweise die Verwendung eines handelsüblichen PCs mit entsprechend angepasster Software möglich. Erfindungsgemäß bevorzugt ist jedoch die Verwendung eines Mikrocomputers, der in verschiedenen Programmier- und Entwicklungsumgebungen (z.B. Arduino, Raspberry Pi, UIFlow (Blockly, Python)) angeboten wird. Mittels einer derartigen Entwicklungsumgebung wird ein einheitliches Systemumfeld erzeugt, welches Steuerung und Auswertung der Messung auf einfache und kostengünstige Weise erlaubt.

Eine integrierte Entwicklungsumgebung (Abkürzung IDE, von englisch integrated development environment) ist eine Sammlung von Computerprogrammen, mit denen die Aufgaben der Softwareentwicklung (SWE) möglichst ohne Medienbrüche bearbeitet werden können.

IDEs stellen hilfreiche Werkzeuge bereit, die dem Softwareentwickler häufig wiederkehrende Aufgaben abnehmen, einen schnellen Zugriff auf einzelne Funktionen bieten, mit denen die Arbeits(zwischen)ergebnisse verwaltet und in spätere Bearbeitungsfunktionen direkt überführt werden können. Der Entwickler wird dadurch von formalen Arbeiten entlastet und kann seine eigentliche Aufgabe, das Entwickeln/Programmieren von Software, mit Systemunterstützung effizient ausführen.

IDEs gibt es für nahezu alle Programmiersprachen und Plattformen. Oft wird damit nur eine Programmiersprache unterstützt. Es gibt aber auch Anwendungen, die mehrere spezielle IDEs unter einer gemeinsamen Benutzeroberfläche zusammenfassen. Auch gibt es sie für Konzepte, die darauf zielen, mehr oder weniger "programmierfrei" Anwendungssoftware per Konfiguration zu erstellen (z. B. Universal Application), und die somit nicht auf eine bestimmte Programmiersprache ausgerichtet sind (Deklarative Programmierung). Insbesondere besteht der Vorteil der Nutzung von IDEs darin, dass alle Systemkomponenten aufeinander abgestimmt sind.

Für den erfindungsgemäßen Einsatz geeignete Mikroprozessoren, Programmier- und Entwicklungsumgebungen hierfür sind beispielsweise
- *STM32F103C8T6 ARM Mini system Development Board STM32 Development Core Board*
- *ESP32-CAM WiFi* + *Bluetooth-Kameramodul-Entwicklungsplatine ESP32 mit Kameramodul OV2640*
- STM32F103C8T6 - 64KB Flash, STM32 ARM Cortex-M3 Mini System Dev.board (STM firmware)
- MSStack-basierte Umgebungen, die in zahlreichen Ausführungsformen kommerziell erhältlich sind

Die Anzeige des Messergebnisses kann quantitativ oder qualitativ erfolgen. Die Anzeige kann im einfachsten Fall durch Lichtsignale (z.B. farbige LEDs) oder ähnliches erzeugt werden. Erfindungsgemäß bevorzugt ist die Anzeige des Messergebnisses auf einem Display in qualitativer und quantitativer Form, beispielsweise in der in EP2870477B1 gezeigten Ampeldarstellung.

Die erfindungsgemäße Vorrichtung enthält darüber hinaus ein lichtundurchlässiges Gehäuse, welches mindestens die oben genannten Komponenten Sender, Funktionsschicht und Empfänger beinhaltet. Das Gehäuse dient insbesondere dazu, die Probe, das heißt die Funktionsschicht, von Störlichtquellen fern zu halten und so eine störungsfreie Messung zu ermöglichen. Dabei sind Ausführungsformen der Erfindung denkbar, bei der Steuerung und/oder Anzeige mit in das Gehäuse eingebaut sind. Alternativ ist denkbar, dass Steuerung und Display getrennt von dem Gehäuse angeordnet sind. Die notwendigen Steuer- bzw. Messsignale können dann entweder durch entsprechende Kabel oder auch kabellos (beispielsweise durch W-LAN, Bluetooth) übertragen werden.

Das Gehäuse enthält Halteelemente zur Aufnahme der Funktionsschicht, die ggf. an die jeweilige Halterung der Funktionsschicht (z.B. Testkassette) angepasst ist.

Wie oben bereits dargestellt, erfolgt die Beleuchtung der Funktionsschicht sowie auch die Messung des reflektierten Lichtes bevorzugt lokal, das heißt an der Stelle, an der das Auftreten der Agglutinationsreaktion zu erwarten ist. Die Messung erfolgt üblicherweise im Vergleich mit einer zweiten Messstelle, die als Kontrolle dient. Hierzu werden in einer bevorzugten Ausführungsform zwei Stellen der Funktionsschicht beleuchtet, nämlich das Messfeld und ein Kontrollfeld.

Auch die Messung des reflektierten Lichtes durch das Empfängermodul erfolgt an denselben Stellen, das heißt im Messfeld und im Kontrollfeld. Wie oben bereits dargestellt, werden daher Sender wie auch Empfängermodule nur an den vorgesehenen Stellen angesteuert.

Zum Zeitpunkt der Messung müssen sich Sender, Funktionsschicht und Empfänger in dem lichtundurchlässigen Gehäuse befinden. Dabei muss eine gleichmäßige Beleuchtung der Messfelder der Funktionsschicht gewährleistet sein. Für den Fall einer lichtundurchlässigen Funktionsschicht ist eine Anordnung bevorzugt, bei der die LEDs ringförmig um das Empfängermodul herum angeordnet sind.

Das System Gehäuse-Sender-Empfänger kann auch nach dem Prinzip einer Ulbricht'schen Kugel angeordnet sein.

Möglich ist, dass die Funktionsschicht nach dem Auftropfen der Probe in das Gehäuse eingelegt wird. Erfindungsgemäß bevorzugt ist, dass die Funktionschicht zum Zeitpunkt des Auftropfens der Probe sich bereits im Gehäuse befindet. Dies ermöglicht eine präzise Bestimmung der Zeit zwischen Auftropfen und Messung. Für diesen Fall muss das Gehäuse eine Öffnung zum Einführen eine Pipette o.ä. enthalten.

Im Rahmen des erfindungsgemäß bevorzugten Messverfahrens werden Mess- und Kontrollfeld nacheinander mit verschiedenen Wellenlängen beleuchtet und das jeweils reflektierte Licht gespeichert. In einer erfindungsgemäß besonders bevorzugten Ausführungsform erfolgt die Messung sequenziell mit verschiedenen Wellenlängen, z.B. 1. Rot, 2. Grün, 3. Blau, 4. Rot, 5. Grün, 6. Blau (2 Messzyklen). Üblicherweise wird der Messzyklus, nämlich die Beleuchtung und Messung mit den verschiedenen Farben mehrfach durchgeführt, insbesondere 2-5 mal).

In einer besonders bevorzugten Ausführungsform der Erfindung werden die Messergebnisse der Blau- und Grünmessungen addiert und von der gebildeten Summe die Rotmessungen subtrahiert. Die so erhaltenen Messwerte des Messfeldes und des Kontrollfeldes werden miteinander verglichen. Wird im Messfeld weniger als ein bestimmter Anteil des Lichtes des Kontrollfeldes gemessen (z.B. weniger als 75%, 60%, 50% oder 40%), wird das Ergebnis als positiv angesehen. Der genaue cut-off der Lichtmessung hängt von verschiedenen Parametern ab (u.a. Art der der Funktionsschicht, Färbung der Schicht und/oder der Probe, Art und Größe der Partikel, etc.) und ist im Rahmen der Kalibrierung festzulegen.

In einer besonderen Ausführungsform der Erfindung wird das Verfahren wie folgt durchgeführt: Es wird ein üblicher Lateral-Flow Teststreifen herangezogen, auf den die Probe an der üblichen Stelle aufgetragen wird. Unmittelbar nach Probenauftrag wird der Teststreifen in die Vorrichtung eingelegt und das Messverfahren gestartet. Die Messung beginnt mit einer ersten Messung des Kontrollfeldes nach dem oben beschriebenen Schema. Nach Auftragen der Probe auf das Probenfeld erfolgt eine Flüssigkeitsverteilung entlang des Teststreifens (Flow), welche zu einer leichten Änderung der Farbe bzw. der Helligkeit des Teststreifens führt. Durch regelmäßige Messung des Kontrollfeldes (z.B. alle 20 Sekunden) kann festgestellt werden, ob und wann die Flüssigkeitsfront das Kontrollfeld erreicht. Auf diese Weise kann optional eine erste Kontrolle erfolgen, ob die Funktionsschicht korrekt befeuchtet wird. Alternativ kann die Benetzung der Funktionsschicht auch durch Messung des elektrischen Widerstandes der Schicht bestimmt werden. Hierzu werden Platinelektroden mit der Funktionsschicht in Kontakt gebracht. Nach Durchlaufen der Flüssigkeitsfront sinkt der Widerstand der Schicht erheblich ab.

Die auf dem Teststreifen ablaufende Agglutinationsreaktion wird nach Ablauf einer bestimmten Zeit gemessen (z.B. nach fünf Minuten). Die Einhaltung der genauen Zeit ist notwendig, weil sonst die Messung verfälscht werden kann. Die eigentliche Messsequenz setzt daher nach Ablauf der vorbestimmten Zeit (z.B. nach fünf Minuten) ein, sofern die oben ausgeführte Kontrollmessung positiv verläuft. Die Messung kann dann in der oben beschriebenen Weise durchgeführt und ausgewertet werden. Das Messergebnis wird auf dem Display angezeigt.

Der besondere Vorteil des oben beschriebenen Messverfahrens liegt darin, dass das Ergebnis unabhängig ist von der subjektiven Wahrnehmungsfähigkeit des menschlichen Auges, sondern dass auch chargenabhängige Farbveränderungen der Funktionsschicht und probenabhängige Farbveränderungen (z.B. durch unterschiedliche Stuhlfarben) kompensiert werden können. Die Kalibrierung durch die erste Kontrollmessung erlaubt eine sichere Eliminierung dieser Einflussfaktoren und somit die präzise Auswertung der Messung.

Die Erfindung kann im Rahmen von Nachweisverfahren von im Prinzip allen Biomarkern verwendet werden, sofern die nötigen Antikörper zur Verfügung stehen. Geeignete Biomarker sind Beispielsweise: Hämoglobin, M2-PK, Calprotectin, Lactoferrin, Helicobacter pylori, Adenoviren, Clostidin dificile, Norovirus, EHEC, Coronaviren, Grippeviren und anderen. Darüber hinaus kann die Erfindung in der forensischen, der umwelt- und der Lebensmittelanalytik eingesetzt werden. Ferner kann die Erfindung für Schwangerschafts- oder Drogenschnelltests verwendet werden. Besonders vorteilhaft ist die Möglichkeit zur Bestimmung zweier oder mehrerer Biomarker, beispielsweise im Rahmen von Drogenscreening oder der gleichzeitigen Bestimmung mehrerer verschiedener Virenstämme.

Selbstverständlich sind Ausführungsformen der Erfindung möglich, bei denen mehrere Funktionsschichten parallel bearbeitet werden. Die verschiedenen Funktionsschichten, z.B. zum synchronen Nachweis von M2-PK und Hämoglobin, können in einer Testkassette vereinigt sein oder in verschiedenen Kassetten. Die gesamte Steuerung kann auch roboterunterstützt sein, so dass das Auftropfen der Probe, Einlegen der Funktionsschicht (mit oder ohne Kassette) in die Vorrichtung, Messung und Entnahme der Funktionsschicht (mit oder ohne Kassette) vollautomatisch erfolgt. Weiterhin können Funktionsschichten auch so konzipiert sein, dass mehrere Tests auf einer Funktionsschicht durchgeführt werden können. Hierzu müssen die festkörperfixierten Antikörper für verschiedene Biomarker an unterschiedlichen Positionen der Schicht fixiert sein. Ferner müssen der Empfänger und der Prozessor zur Steuerung und Signalbearbeitung so abgestimmt sein, dass mehr als eine "Region of Interest" (ROI) gemessen und ausgewertet wird.

In einer alternativen Ausführungsform der Erfindung erfolgt die Detektion der Antigen-Antikörperreaktion durch entsprechende Fluoreszenzmarker. In diesem Fall wird die Messung ganz analog zur oben geschilderten Messung durchgeführt. Zu berücksichtigen ist lediglich, dass das die Wellenlänge des gemessenen Lichtes von der Wellenlänge der Lichteinstrahlung verschieden ist. Die Fluoreszenz wird durch den Empfänger detektiert und das Ergebnis auf dem Display angezeigt.

In einer weiterhin alternativen Ausführungsform der Erfindung erfolgt die Detektion der Antigen-Antikörperreaktion durch Chemilumineszenz. In diesem Fall wird der Sender nicht als Lichtquelle benötigt. Die Chemilumineszenz wird durch den Empfänger detektiert und das Ergebnis auf dem Display angezeigt.

### Beispiele:

Weitere Erläuterungen werden durch die nachfolgenden Beispiele gegeben. Die nachfolgenden Beispiele zeigen, wie die Erfindung ausgeführt werden kann, ohne sie auf diese speziellen Ausführungsformen beschränken zu wollen. Der Fachmann auf dem Gebiet kann auf Grundlage dieser Beispiele und der weiteren Beschreibung weitere Ausführungsformen der Erfindung erarbeiten ohne erfinderisch tätig werden zu müssen.

### Messaufbau: Controller

Grundsätzlich kann jeder Microcontroller (8/16/32Bit) verwendet werden, welcher über ausreichend Flash/ RAM - Speicher verfügt (die Organisation des Speichers spielt keine Rolle) und genügend freie IO's zur Kamerasteuerung sowie für die restlichen Steueraufgaben besitzt, ebenso muss er die Signale des Kameramoduls (8bit synchroner parallel Port) in der nötigen Geschwindigkeit verarbeiten können. Als Beispiele wären von Microchip die ATMega/ATXMega Reihe (vormals Atmel), die PIC Serie, von diversen Herstellern die Cortex-Serien, von Parallax der Propeller, nur um Beispielhaft ein paar Hersteller / Typen zu nennen. Vorzugsweise wird ein STM32F103 verwendet (C8T6, 72MHz, 32Bit, 20kB Ram, 64kB Flash).

Die Entscheidung auf den STM32F103 fiel auf Grund der guten Verfügbarkeit und guter Softwareunterstützung seitens des Herstellers sowie vieler verfügbarer Beispiele.

Programmiert ist der Microcontroller in C/C++, es ist möglich eine beliebige Programmiersprache einzusetzen, welche eine hardwarenahe Programmierung zulässt. Um für die weitere Entwicklung möglichst flexibel zu bleiben, wurde eine Entwicklungsumgebung ausgewählt, welche eine Prozessorplattform-übergreifende Entwicklung ermöglicht und eine möglichst gute Unterstützung im Internet in Sachen Beispielcodes bietet.

### Sender (Lichtquelle)

Zur Beleuchtung dienen LED RGB Module (z.B. Ansteuerbaustein WS2812). Bei diesen Modulen lassen sich relativ einfach mittels eines seriellen Protokolls (onLine) die Farben pixelweise einzeln oder auch als Mischfarben ansteuern. Für andere Messzwecke ist es auch möglich, LEDs, welche andere Wellenlängen emittieren (z.B. IR oder UV), zu verwenden. In diesem Fall ist gegebenenfalls ein Farbfilter für das Kameramodul nötig. Für die Ansteuerung muss ein entsprechender Schaltungsblock zusätzlich vorgesehen werden.

Betrieben wird der komplette Aufbau über einen 5V Step-up Regler aus 2AA-Zellen. Es hat sich bei Tests herausgestellt, dass eine Versorgung aus 4 in Serie geschalteten AA-Zellen zu große Spannungsschwankungen hat, ein nachgeschalteter Linearregler grenzt den zur Verfügung stehenden Kapazitätsbereich der Batterien stark ein. Ein geschalteter StepUp/StepDown Regler wurde auf Grund der Komplexität als weniger bevorzugt eingeschätzt.

### Empfänger

Das Kameramodul ist ein OV7670 (Omnivision) mit eingebautem Signalprozessor, die maximale Auflösung beträgt 640x480pixel. Angesteuert wird das Modul durch den SCCB (serial Camera Control Bus) vom Microcontroller aus. Grundsätzlich kann jedes Kameramodul verwendet werden welches die nötigen Daten zur Verfügung stellt

### Auswertung

Für die Auswertung wird nur ein kleines Fenster (ca.64x16Pixel und ca. 16x16Pixel als Referenz) von der möglichen Kameraauflösung verwendet, ebenso wird nur das Y Signal (Helligkeit) des YUV Signals verwendet. Die Daten aus den Farbinformationen U und V werden nicht ausgewertet.

Es wäre auch möglich, ein monochromes Kameramodul einzusetzen. Das eingesetzte Kameramodul muss nur die nötige Empfindlichkeit in dem zu detektierenden Wellenlängenbereich besitzen.

Im Anschluss der Messung steht der ermittelte Wert zur Weiterverarbeitung zur Verfügung, dieser kann z.B. über Bluetooth oder USB an einen PC oder Smartphone weitergegeben werden oder nach einer Klassifizierung auf einem kleinen Display angezeigt werden.

### Messprinzip

Nach einer Wartezeit (beispielsweise nach etwas weniger als 5min, damit nach genau 5min gemessen werden kann, dies ist abhängig von der Probe) erfolgt eine Kontrolle der Beleuchtungsstärken der einzelnen Lichtquellen mit den Sollwerten.

Hierzu wird der Messstreifen mit den einzelnen Lichtquellen nacheinander beleuchtet. Örtlich angrenzend an das normale Messfenster befindet sich noch ein kleineres Messfenster (ca. 16x16 Pixel), welches dazu dient, nachdem über alle Pixel des kleinen Messfensters der Median gebildet wurde, die Ist - Beleuchtungsstärke auf einen Wert +/-1 zu dem Soll - Wert anzugleichen.

Anschließend wird der Messstreifen nacheinander mit der Lichtquelle in den unterschiedlichen Wellenlängen beleuchtet und jeweils die Helligkeitsinformationen, welche durch die unterschiedliche Reflektionen/Absorbtionen in den einzelnen Wellenlängenbereichen her rührt, der einzelnen Pixel des großen Messfensters (ca.64x16 Pixel) gespeichert. Aus diesen Informationen wird spaltenweise der Median gebildet und die Ergebnisse in einer einzeiligen Tabelle gespeichert.

Dieser Ablauf wird mehrfach wiederholt, aus jedem Durchlauf wird spaltenweise der Median gebildet und zu dem spaltenweise schon vorhandenen Wert hinzuaddiert. Der niedrigste Wert der Zeile ergibt noch einen Korrekturfaktor, welcher von allen Werten der Tabelle subtrahiert wird. Durch dieses Verfahren ist es möglich, differenziertere Messergebnisse zu erhalten. Ebenso wird das große Messfenster bei jedem Durchlauf um 1 Pixel (1 Zeile) verschoben, wodurch kleine Ungleichmäßigkeiten, welche bei der Produktion der Teststreifen entstehen, messtechnisch verringert werden. Es hat sich bei Betrachtung unter dem Mikroskop gezeigt, dass die in der Produktion der Teststreifen aufgebrachten "Goldfallen" längst nicht so gleichmäßig sind wie gedacht.

Nachdem alle Durchläufe der Farben abgeschlossen sind (typischerweise nach 2-5 Durchläufen), werden die Spaltenwerte von der Farbe, welche als Referenzfarbe dient (d.h. die Testpartikel beeinflussen diese Lichtfarbe nicht oder nur in geringem Maße) von den Spaltenwerte der anderen Beleuchtungsfarben subtrahiert, um Störeinflüsse wie Streulicht, Verschmutzung, ungleichmäßige Beleuchtung und Unzulänglichkeiten des Kameramoduls, auszugleichen.

Anschließend werden die so gewonnenen Spaltenwerte der für die Messung relevanten Farben addiert. Nun wird von allen Werten das arithmetische Mittel gebildet, um das Grundrauschen heraus zu rechnen. Der eigentliche Messwert wird durch Bildung des Integrals der Spaltenwerte oberhalb des arithmetischen Mittels gebildet.

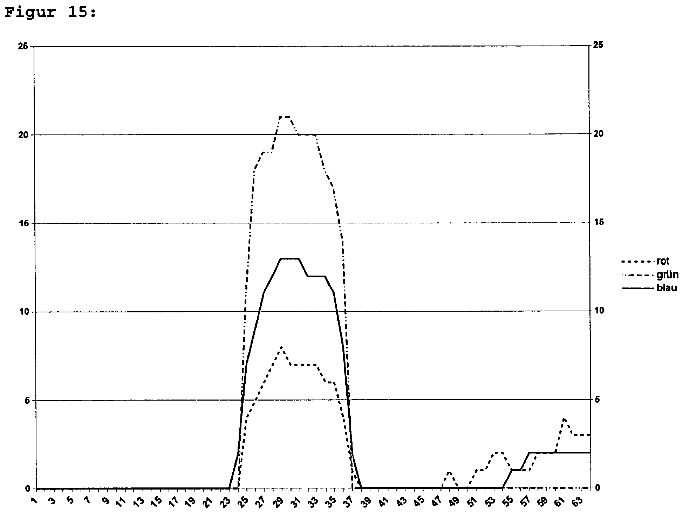

Die vor der Messung durchgeführte Kalibrierung der Lichtquelle(n) ist unbedingt nötig, bei Tests hat sich ein überraschendes Verhalten der des Kameramoduls bezüglich der Höhe der Messwerte des Messstreifens gezeigt. Dies äußert sich in einem stark unlinearen Zusammenhang zwischen Beleuchtungsintensität und Messergebnis bei zunehmender Beleuchtungsintensität (siehe Figur 15). Da der Grad der maximalen Empfindlichkeit relativ schmal ist, ist eine genaue Kalibrierung notwendig.

Das Kameramodul besitzt für die Einstellungen von unter anderem Kontrast, Helligkeit, Farben und Belichtungszeit viele Register in denen diese Parameter eingestellt werden können, viele von diesen Einstellungen wurden für diesen Messzweck speziell angepasst.

Die Medianberechnung wird mit einem Algorithmus nach N.Wirth berechnet, dieser stellt einen guten Kompromiss zwischen der Codelänge, dem Speicherverbrauch während der Berechnung und der Geschwindigkeit dar.

### Messdetails

Das von den LEDs abgestrahlte Licht ist relativ schmalbandig mit Peak bei:

| | |
|---|---|
| LED WS2812 | rot 620-630 nm |
| | grün 515-530nm |
| | blau 465-475nm |

Das Absorptionsmaximum der Gold NP 20nm liegt bei 519-520 nm und somit im Peak der grünen LED, blau wird auch zu einem bedeutenden Teil absorbiert, rot nur zu einem geringen Teil (siehe http://www.sciencedirect.com/science/article/pii/ S0925400515307711)

Daraus hat sich folgende Messsequenz als sinnvoll herausgestellt:
Beleuchtung des Messobjektes nacheinander mit den drei Grundfarben erzeugt durch LEDs der Farben rot, grün und blau, für jede Farbe wird ein Bild generiert.

Eine Bildsequenz besteht aus 3 Aufnahmen (jeweils 1 Aufnahme beleuchtet mit rot, eine mit grün und eine mit blau) daraus wird spaltenweise ein Median gebildet um Störungen, Pixelfehler und Rauschen zu minimieren.

Es werden 16 Bildsequenzen aufgenommen, jede besteht aus 3 Aufnahmen in den Farben rot, grün und blau, also 48 Aufnahmen gesamt. Der jeweilige Median der einzelnen farblich zusammengehörigen Aufnahmen wird zeilenweise zwischengespeichert.

Nach Abschluss des kompletten Messdurchlaufes wird aus den gespeicherten Zeilen farblich zusammengehörig jeweils wieder spaltenweise der Median berechnet.

### Herausrechnen von niederfrequenten Anteilen und des DC Anteils

Die Messwerte welche durch die rote Beleuchtung gewonnen wurde wird von den Werten der blauen und grünen Beleuchtung subtrahiert um Staub und Dreck nochmals zu reduzieren (Nebeneffekt: das Rauschen wird nochmals etwas reduziert)

Abschließend werden die Werte von Blau und Grün addiert, der DC Anteil und eventuell noch vorhandene niederfrequente Störungen herausgerechnet und das Integral der Werte, welche sich über dem arithmetischen Mittel befinden, gebildet. Dieser Wert wird als Messergebnis ausgegeben bzw. weiterverarbeitet zu einer verständlicheren Anzeigeform.

### Beispiel 1: Unterschiedliche Ausführungsformen

Die Bestimmung der humuralen Immunantwort eines Probanden verursacht durch eine Infektion mit Corona-Viren, insbesondere mit dem SARS-CoV-2-virus, ist eine besondere technische Herausforderung.

Informationen bzgl. einer eventuell vorhanden humuralen Immunantwort bzgl. der in der Probe vorhandenen Antikörper, die gegen diese Viren-Klassen-Antigene gerichtet sind zu erhalten. Aber besonders wichtig Informationen der Antikörper, die gegen die Antigene des SARS-CoV-2-virus gerichtet sind zu erlangen. Klinisch und diagnostisch ist es von besonderer Wichtigkeit Informationen bzgl. der Anti-Körper-Population zu erhalten. Besonders bezgl. der Fähigkeit dieser Antikörper das Virus zu binden, zu inaktivieren bzw. die Heftigkeit der Virusinfektion, via Antikörperbindung zu mildern.

### Technische Lösung

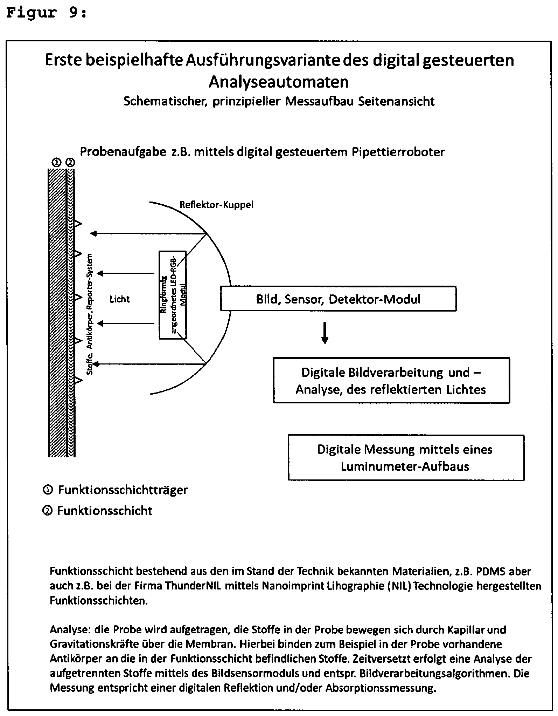

### Diese beispielhafte Ausführungsvariante des digital gesteuerten Analyseautomaten wird in Figur 9 dargestellt

Die Messung / Bestimmung der Anti-Körper-Population (AKP (AKbindungsfähig gegen bestimmte Virus-Antigene)) wird technisch gelöst durch Auftragen der Probandenprobe auf z.B. der speziell vorbereiteten Funktionsschicht, die vertikal oder horizontal (bevorzug vertikal) angeordnet ist.

Der erfindungsgemäß digital gesteuerte Analyse-Vollautomat pipettiert mittels eines Pipettier-Roboters das Probengemisch an den Start. Das Probengemisch bewegt sich mittels Kapillar- und Gravitations-Kraft durch die offenporige Funktionsschicht (also von oben nach unten).

In der Funktionsschicht sind linear oder durch Spots (Flecken) unterschiedliche Fänger-Moleküle (bevorzugt die Verwendung von Rekombinant- Gentechnisch hergestellten Virusantigenen aber auch gentechnisch hergestellten zellulären Molekülen bevorzugt z.B. das für die Virus-Bindung an die Zelle verantwortliche zelluläre-Virus-Rezeptor-Antigen) in der offenporigen Funktionsschicht fixiert und zwar in Flussrichtung von oben nach unten spotförmig oder leiterförmig angeordnet.

Die zu untersuchende Flüssigkeit, enthaltend unterschiedliche Immun-Globuline bindungsfähig für Virusantigene (auch zusammengesetzt aus Immun-Globulin-Klassen) bewegen sich über / in der 3-D-Struktur der offenporigen Funktionsschicht.

Die erfindungsgemäß in der Funktionsschicht an unterschieden Orten (die ortsgebundenen Fängermoleküle) fangen aus dem zu untersuchenden Stoffgemisch die für diese Fängermoleküle bindungsfähigen Antikörper aus der Probe (Stoffgemisch).

Nach einem oder mehreren Wasch-Schritten gem. Stand der Technik erfolgt die qualitative und qualitative Bestimmung der z.B. Antigene mittels nach dem im Stand der Technik bekannten immun-chemischen Verfahren.

Die Detektion erfolgt, wie im Stand der Technik beschrieben, durch Zugabe von Reagenzien (nach einem programmierten Zeitprotokoll) im Stand der Techniken Reporter-Detektions-Aufbauten (Immun-Histochemie), ELISA-Immun-Chemischen-Techniken, Fluoreszents-, Chemoluminiszenz-Techniken. Die "leiterförmige Anordnung" der in der Funktionsschicht befindlichen Fängermoleküle in Flussrichtung (d.h. z.B. von oben nach unten) bewirkt, dass die im Probengemisch vorhandenen unterschiedlichen Antikörper nach und nach (zeitlich) in Flussrichtung im chromatografischen Absorbtionsprozess zeitlich versetzt aus dem Probengemisch entfernt werden, z.B. die leiterförmige Anordnung (von oben nach unten) ist z.B. A) Rekombinantes-Zelluläres-Virus-Rezeptor-Antigen, B) das Rekombinathergestellte-Virus-Spike-Protein (S1), C) Virus-Hüll-Protein (E), D) Virus-Membran-Protein (M), E) Virus-Nukleokapsid-Protein (N) [siehe Abbildung]. Die in der Probe befindlichen Antikörper, die durch die Fängerschicht gebunden entfernt wurden, werden durch Zugabe von sekundären Antikörpern mittels dem Stand der Technik bekannten Reporter immunchemischen Aufbauen qualitativ und quantitativ nachgewiesen.

Die opto-elektronische erfolgte Messung mittels des erfindungsgemäßen Aufbaus der Vorrichtungen erfolgt, wie beschrieben, mit dem erfindungsgemäßen Messverfahren.

### Beispiel 2:

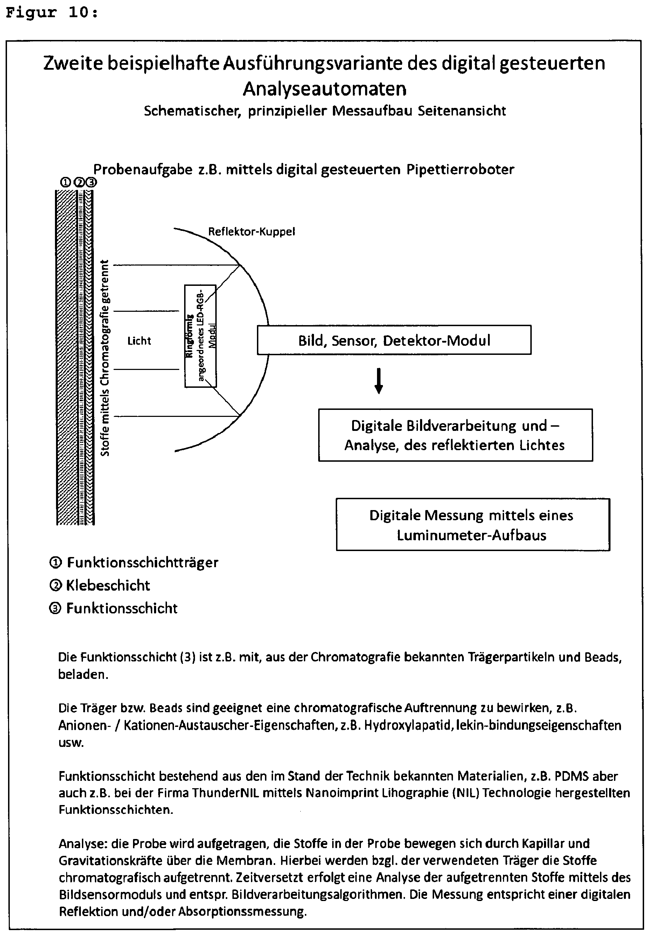

Die zweite beispielhafte Ausführungsvariante des digital gesteuerten Analyseautomaten wird in Figur 10 dargestellt.

Der Ablauf der Messung mittels der Vorrichtungen ist nachfolgend dargestellt

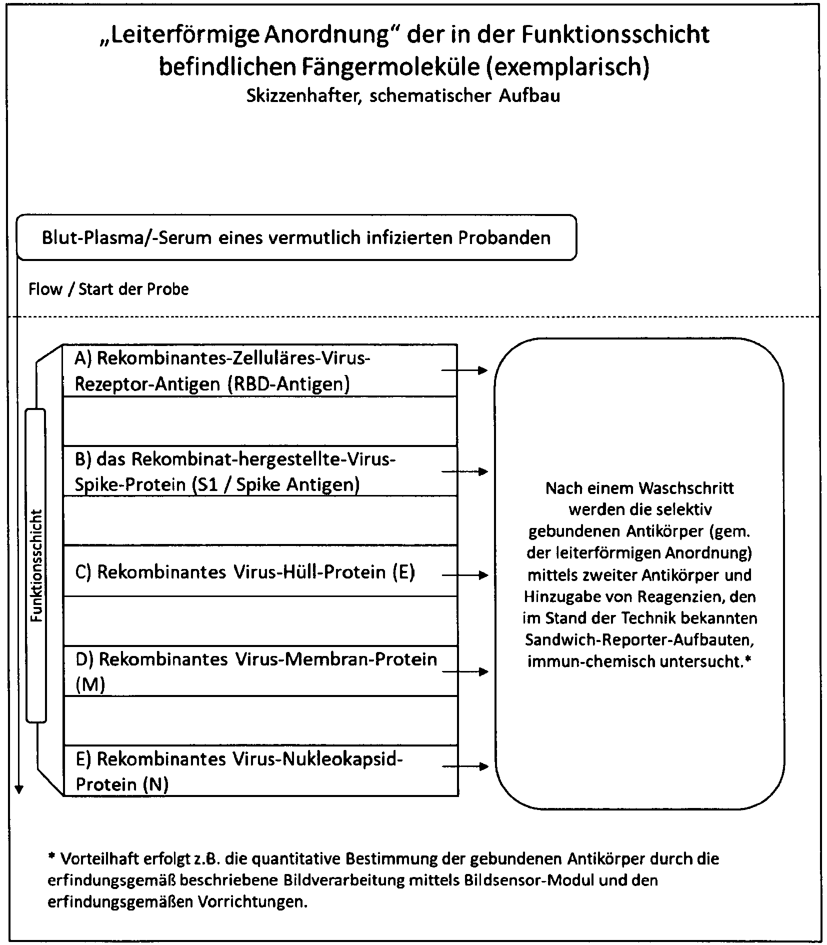

Durch die "chromatographischen" Absorptionsprozesskette werden aus dem Antikörpergemisch, die Antikörper, die bindungsfähig für die jeweiligen Fängermoleküle sind, zeitlich versetzt, aber auch von oben nach unten, selektiv aus dem Probengemisch (Antikörperpopulation) entfernt. Eine qualitative / quantitative, durch digitale Bildbearbeitung ausgewertete Messung, ergibt wichtige Informationen bzgl. der Antikörper-Zusammensetzung in der Probe.

Wie neue Studienergebnisse zeigen sind Antikörper, die gegen das zelluläre RBD-Antigen und / oder gegen das Virus S1 Spike-Antigen gerichtet sind, von besonderer klinischer Wichtigkeit. Diese Information ist wichtig bezüglich des individuellen Infektionsverlaufes durch eine mögliche erneute Infektion durch dasselbe SARS-CoV-2-Virus. Dies ist wichtig bzgl. der Prognose eines möglicherweise Neuinfektionsgeschehens. Durch eine ErstInfektion wird eine Immunabwehr des Probanden erzielt. Die selektive Bindung dieser spezifischen Antikörper an die Virus-Antigene unterdrückt den Eintritt des Viruspartikels in die zu infizierende humane Zelle, z.B. Lungenzelle.

Sind in einer Probandenprobe diese wichtigen, spezifischen bindungsfähigen Antikörper in besonders hoher Konzentration vorhanden, so ist der Proband bzgl. des Infektionsverlaufes nach einer möglichen Neuinfektion durch dasselbe Virus erheblich geschützt.

Diese RBD-Antikörper sind Virus-spezifische Inhibitoren, denn diese Antikörper blockieren, durch die Bindung des Viruspartikel (des Virus-Spike-Proteins an den virus-spezifischen zellulären Rezeptor) und verhindern somit den Eintritt des Viruspartikels in die neu zu infizierende humane Zelle, z.B. Lungenzelle (Schlüssel-Schloss-Prinzip).

Praktisch gesehen: die leiterförmige Anordnung der Fängermoleküle in der Funktionsschicht in Flussrichtung ermöglicht die qualitative und quantitative Vermessung der Antikörperkonzentrationen mittels des erfindungsgemäßen digitalen Messaufbaus.

Ergibt z.B. eine Messung eine Konzentration von Antikörpern, die gegen das zelluläre-Virus-Rezeptor-Antigen (RBD) gerichtet sind und / oder eine hohe Konzentration von Antikörpern, die gegen das Virus-Spike-Protein gerichtet sind, dann hat der Proband eine gute humurale Immunantwort, bedingt durch seiner Erstinfektion generiert.

### Beispiel 3:

In diesem Beispiel werden beispielhafte Einzelkomponenten des erfindungsgemäßen Systems beschrieben.

### Sender: Die Beleuchtungsvorrichtung ist bevorzugt z.B. ein sogenanntes 3-Farben-RGB-SMD-Modul

"3" steht für die Anzahl der LEDs, diese sind jedoch als RGB in SMD Bauweise ausgeführt. Das Modul wird digital angesteuert, sowohl in der Leuchtstärke (Lux) als auch in der spektralen Zusammensetzung des Lichts. Hierdurch lassen sich in etwa 16 Millionen Lichtstrich-Farben erzeugen, Farbtemperatur (Kelvin), Lichtintensität (Lux) digital ansteuern und erzeugen.

Aus dem Stand der Technik sind derartige Module (Power-Module) bekannt. Zum Beispiel werden diese Module zur optimalen Beleuchtung von Pflanzen mit dem Ziel ein optimiertes Pflanzenwachstum zu erreichen, verwendet. Das mittels der Beleuchtungsvorrichtung abgestrahlte Licht ist, in seiner spektralen Zusammensetzung, auf die biophysikalischen bezüglich der Absorptionseigenschaften der Blattfarbstoffe optimiert abgestimmt.

### Technische Herausforderung

Qualitative und quantitative Messung der Stoffe in der Funktionsschicht. Dies wird technisch gelöst durch die Verwendung einer der für diese Stoffe optimalen Beleuchtungsvorrichtung.

Die Lösung: verwendet werden gem. der technischen Aufgabe digital ansteuerbare RBG-LED-Module (Sender). Die Module sind vorzugsweise in SDM-Bauweise gefertigt. Durch erfindungsgemäß ausgewählte Microprozessoren werden diese RGB-Module digital angesteuert. Hiermit ist es technisch möglich, Licht, d.h. ein Gemisch aus elektromagnetischen Wellen unterschiedlicher Frequenzen zu erzeugen. Des Weiteren erzeugen diese erfinderisch ausgewählten LED-Module unterschiedliche Lichtintensitäten und Lichtimpulse.

Die Anordnung des LED-Moduls bezgl. der Funktionsschicht ist so angeordnet, dass die Beleuchtungsvorrichtung die gesamte Funktionsschicht mit Licht beleuchtet/bestrahlt. Das reflektierte bzw. absorbierte Licht wird mittels eines digitalen Luminometer-Messaufbaus insb. unter Verwendung eines Bildsensor-Moduls (Detektor, Empfänger), durch Bildverarbeitungs-Algorithmen aufgearbeitet und analysiert.

Das abgestrahlte Gemisch aus elektromagnetischen Wellen ist hinsichtlich dessen Frequenzen (Lichtspektrum) und Intensität auf die Absorptionseigenschaften der zu messenden Stoffe der Funktionsschicht optimal abgestimmt (siehe oben, Beispiel: Absorptionseigenschaften Blattfarbstoffe). So hat sich zum Beispiel ergeben, dass das in der Funktionsschicht verwendete collidiale Gold (Gold-Nanopartikel 20nm) an die die Antikörper gebunden sind "grünes Licht" (d.h. Lichtgemisch mit einer "grünen spektralen Zusammensetzung" bevorzugt absorbiert). Das Gleiche gilt für "blaues Licht" (d.h. Lichtgemisch mit einer "blauen spektralen Zusammensetzung" auch absorbiert). Testreihen haben ergeben, dass die Funktionsschicht selbst "rotes Licht" (d.h. Lichtgemisch mit einer "roten spektralen Zusammensetzung" nicht absorbiert, sondern reflektiert).

Das jeweilige "rote Licht", "grüne Licht" und "blaue Licht" welches von der Beleuchtungsvorrichtung abgestrahlt wird ist so durch Testreihen optimiert worden, dass eine flächenbezogene Absorptions- bzw. Reflektions-Messung mittels eines digitalen Bildsensors optimal ermöglicht wird.

Überraschenderweise wurde bei Testungen gefunden, dass die zeitliche Abfolge der Beleuchtung der Funktionsschicht mittels der Beleuchtungsvorrichtung zum Zwecke der optimalen Messaufgabe optimiert werden kann. Hierbei wurde gefunden, dass eine ortsbezogene Vermessung mittels Bildsensor zu einem besseren Messergebnis führt.

Die digitalen Bildbearbeitungs-Algorithmen erlauben selektiv das abgestrahlte Licht der Funktionsschicht ortsaufgelöst zu vermessen. Besondere Orte in der Funktionsschicht, die Vermessen und Errechnet werden Rol (Regions of Interest) bezeichnet.

### Beispiel 4:

In diesem Beispiel wird beispielhaft die digitale Bildverarbeitung des erfindungsgemäßen Systems beschrieben:
1. Messung der Flächen-ROI (Regions of Interest) insbesondere der Erwartungsregion (Testlinienregion) mit einer optimierten "roten spektralen Licht-Zusammensetzung".
2. Messung: Messung der Flächen-ROI (Regions of Interest) insbesondere der Erwartungsregion (Testlinienregion) mit einer optimierten "grünen spektralen Licht-Zusammensetzung".
3. Messung: Messung der Flächen-ROI (Regions of Interest) insbesondere der Erwartungsregion (Testlinienregion) mit einer optimierten "blauen spektralen Licht-Zusammensetzung".

Die in zeitlicher Beleuchtungs-Abfolge ist erfindungsgemäß bezüglich dessen Intensität und Farbzusammensetzung (siehe oben) microprozessor-gesteuert. Die digitale Vermessung erfolgt mittels eines Bildsensor-Moduls. Die Messung, d.h. die Erzeugung der digitalen Bilddaten erfolgt durch digitale Abtastung (Sampling) durch Quantisierung der Bildmatrix der einzelnen Bildpunkte (Pixel). Die einzelnen digitalen Intensitätswerte (digitale Helligkeitswerte) der Bildpunkte / Pixel werden quantisiert und durch digitale Messtechnik, (Signalverarbeitung) in numerische Werte überführt und zum Beispiel in einem Display angezeigt oder via Schnittstelle zu einem PC übertragen.

Die digitalen Messergebnisse Ergebnisse der Quantifizierzung der Bildmatrix der einzelnen Bildpunkte der ROI werden nach jeder Messung digital in einem Speicher auf dem Microprozessor-Board eingebracht, jeweils für die Messung der Schritte 1., 2. und 3.

Des Weiteren werden die Ergebnisse der einzelnen Messung bezüglich der ortbezogenen Messung die bei der ortbezogenen Messung erhalten werden gegeneinander verrechnet und in einem Speicher auf dem board abgelegt. Die digitalen Informationen der einzelnen Messung werden aus dem Speicher ausgelesen und mit digitalen Verarbeitungsalgorithmen, die im Stand der Technik bekannt sind verrechnet. Bei diesen digitalen Verrechnungen kommen Smoothing-Algorithmen, insbesondere die Verwendung des digitalen K-means-Clustering und anderen digitale Rechenverfahren erfindungsgemäß zum Einsatz.

Insbesondere hat sich gezeigt, dass das sogenannte Stochastic-Neighbor-Embedding (SNE) (d.h. ortsbezogene, pixel-bezogene Messungen) digitales Auswertungs-Verfahren zu einem optimierten Messergebnis führt.

Da die Gesamtmessung, wie oben beschrieben, durch drei zeitlich nacheinander ablaufende Messungen erfolgt, werden erfindungsgemäß die Stochastic-Time-Embedding (STE) (d.h. zeitbezogene Messungen) digitales Auswertungs-Verfahren zu einem optimierten Messergebnis führt.

Im Bildsensor-Modul wurde durch entsprechende Programmierung, die für die Messung entscheidenden ROI (ortsbezogen), festgelegt. Die Erwartungsregion (Testlinien-Region) z.B. in einer Lateral Flow Membran, ist eine ROI. Eine weitere ROI, z.B. auf einer Lateral Flow Membran ist eine Region zwischen der Testlinien Region und der Kontroll-Linien-Region (d.h. eine Region an der kein durch Antikörper bewirktes Immun-Präzipitat vermittelt durch kolloidales Gold zu erwarten ist).

Diese Makro-ROI ("Testlinien und Blank-Regionen") sind zu unterscheiden zu den o.g. Micro-ROI (Pixel). Die Pixel ROI sind wesentlich kleiner, d.h. z.B. die ROI in der Testlinien-Region sind circa 50 Mal größer.

Diese gemessene Antikörper-Zusammensetzung ermöglicht höchstwahrscheinlich in diesem Fall die Prognose eines guten Infektionsverlaufes bei einer Neuinfektion mit dem Virus.

### Beispiel 5:

Eine erfindungsgemäße Vorrichtung besteht aus einem lichtundurchlässigem Gehäuse und enthält eine Haltevorrichtung zum Fixieren einer handelsüblichen Testkassette für die dimere Form des Tumormarkers M2PK. Diese enthält eine Funktionsschicht (auf Nitrocellulosebasis) mit bekannten Antikörpern zum immunologischen Nachweis der dimeren Form des Tumor M2PK. Oberhalb der Testkassette befindet sich ein Sender bestehend aus einem LED-Modul Typ WS2812. Das Licht der LEDs diesen Typs ist relativ schmalbandig mit Peaks bei 620 bis 630 nm (rot), 515 bis 530 nm (grün) und 465 bis 475 nm (blau). Ferner enthält die Vorrichtung ein Kameramodul OV7670 mit eingebautem Signalprozessor. Dieses Kameramodul weist eine Auflösung von 640 x 480 Pixel auf, von denen allerdings nur ein Teil ausgewertet wird (siehe unten). Die Vorrichtung wird gesteuert von einem Mikrocontroller des Typs STM32F103 (C8T6, 72 MHz, 32 Bit, 20 kB RAM, 64 KB Flash). Ferner enthält die Vorrichtung ein digitales Display zur Anzeige des Messergebnisses. Nach dem Einlegen der Testkassette und dem Verschließen des Gehäuses erfolgt das Auftropfen der Probelösung auf die dafür vorgesehene Öffnung der Testkassette. In bekannter Weise bewegt sich die aufgetropfte Flüssigkeit durch die Funktionsschicht in Richtung auf das Wick der Testkassette. Gleichzeitig beginnt die Zeitmessung (Auftropfen: t = 0). Nach Ablauf der vorgesehenen Testzeit werden die LEDs angesteuert. Der Teil der Testkassette, an der die Messlinie erwartet wird (Region of Interest, ROI) wird mittels des Kameramoduls in einer Auflösung von ca. 64 x 16 Pixel aufgenommen. Diese Region of Interest wird nacheinander mit den drei Grundfarben, erzeugt durch die geschilderten LEDs beleuchtet, das heißt jeweils mit den Farben Rot, Grün und Blau beleuchtet. Für jede dieser Farben wird ein Bild generiert. Eine Bildsequenz besteht daher aus drei Aufnahmen, nämlich jeweils eine Aufnahme beleuchtet mit Rot, Grün und Blau. In jeder Aufnahme wird spaltenweise ein Median gebildet, um Störungen, Pixelfehler unter Rauschen zu minimieren. Nacheinander werden insgesamt 16 derartiger Bildsequenzen aufgenommen, jede besteht aus drei Aufnahmen. Es werden somit insgesamt 48 Aufnahmen generiert. Der jeweilige Median der einzelnen farblich zusammengehörigen Aufnahmen wird zeilenweise zwischengespeichert. Nach Abschluss des kompletten Messdurchlaufes wird aus den gespeicherten Zeilen farblich zusammengehörig jeweils wieder spaltenweise der Median berechnet. Anschließend werden niederfrequente Anteile und der DC-Anteil herausgerechnet. Die Messwerte, welche durch die blaue und grüne Beleuchtung gewonnen wurde, werden addiert, von der Summe werden die Messwerte, die durch die rote Beleuchtung gewonnen wurden, subtrahiert. Auf diese Weise werden Messartefakte durch Staub und Dreck reduziert, darüber hinaus wird das Rauschen nochmals reduziert. Abschließend wird das Integral der Werte, welche sich über dem arithmetischen Mittel befinden, gebildet. Dieser Wert wird als Messergebnis ausgegeben bzw. weiterverarbeitet zu einer verständlicheren Anzeigeform.

In einer weiter optimierten Vorrichtung wird zusätzlich in exakt derselben Weise eine weitere Region der Funktionsschicht als Referenz aufgenommen und auf die oben beschriebene Weise ausgewertet. Durch den Vergleich der Messung der Region of Interest mit dem Referenzfeld wird die Messung weiter präzisiert. Für die Messung der Referenzregion hat sich ein Messfeld von 16 x 16 Pixel als ausreichend erwiesen.

In einer weiteren Variante der Erfindung wird die Vorrichtung mit einer Kombitestkassette versehen, welche einen kombinierten Test M2PK und Hämoglobin enthält, so wie er in US 2015/0219658A1. beschrieben ist. In diesem Fall muss gleichzeitig eine Auftragung der Probelösung auf zwei Stellen der Funktionsschicht erfolgen, sodass zwei Pipetten nötig sind. Die Auftragung kann sowohl manuell als auch robotergesteuert erfolgen.

Die Messung und Auswertung dieses Testsystems erfolgt ganz analog zur oben beschriebenen Messung und Auswertung. Allerdings werden hier zwei voneinander verschiedene Regions of Interest ausgewertet, nämlich eine Region of Interest für die M2PK, die andere Region of Interest für Hämoglobin.

### Beispiel 6:

Anwendungsbeispiele für Messungen im Lateral Flow Format Kassetten:
1) ScheBo Elastase (Einzel-Kassette / eine lateral-flow Laufstrecke)
2) ScheBo M2PK Stuhl (Einzel -Kassette / eine lateral-flow Laufstrecke)
3) ScheBo 2in1 (Doppel-Kassette / zwei lateral-flow Laufstrecken)
4) ScheBo M2PK Urin (Einzel -Kassette / eine lateral-flow Laufstrecke)
5) ScheBo SARS-CoV-2 (Einzel -Kassette / eine lateral-flow Laufstrecke) Antikörper, die gegen Virus-Antigene gerichtet sind. Der Test dient der Bestimmung der Erzielung einer Information bezüglich der humoralen Immunantwort des menschlichen Körpers auf eine eventuell erfolgte Infektion des Körpers mit SARS-CoV-2 Viren.
6) ScheBo SARS-CoV-2 (Einzel -Kassette / eine lateral-flow Laufstrecke) Messung von SARS-CoV-2-Antigen und/oder - Antigenen. Zur Bestimmung von Virus-Antigenen im menschlichen Körper, bewirkt durch eine eventuell vergangene Infektion des Körpers mit SARS-CoV-2 Viren.

### Beispiel 7a:

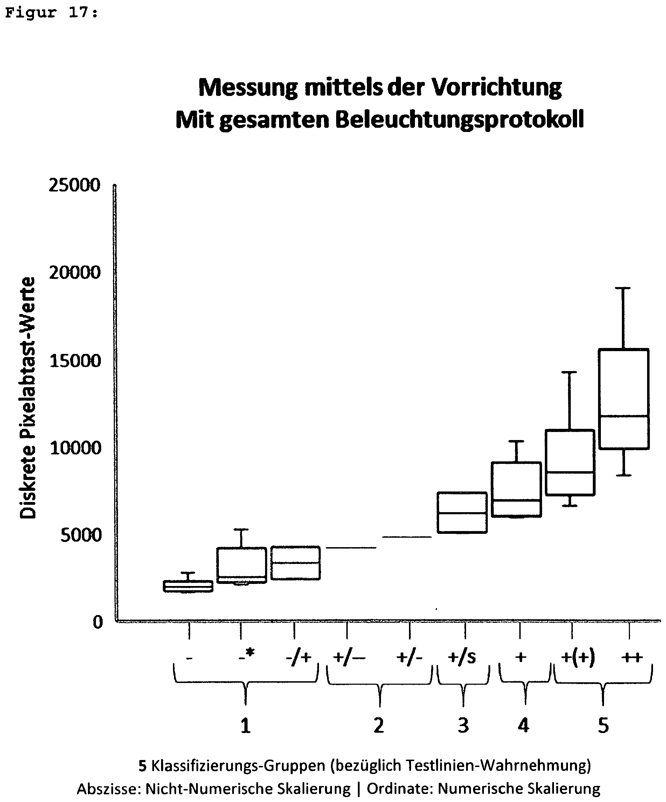

Es wurde eine Biomarker-Konzentrations-Bestimmung (Tumor - M2PK) in Probanden-Stuhlproben mit einer herkömmlichen M2-PK-Einzel-Testkassette durchgeführt. Die Auswertung erfolgte auf herkömmlichem Wege durch 3 beruflich mit derartigen Tests befassten Personen
Visuelle Wahrnehmung der Testlinien vs. digital-optischer Vermessung der Testlinien
Das Messergebnis ist in Figur 17 dargestellt
5 Klassifizierungs-Gruppen (bezüglich Testlinien-Wahrnehmung)
Abszisse: Nicht-Numerische Skalierung | Ordinate: Numerische Skalierung

| | |
|---|---|
| Gruppe 1 = Testlinien fürs Auge | nicht wahrnehmbar |
| Gruppe 2 = Testlinien fürs Auge | sehr schwach wahrnehmbar |
| Gruppe 3 = Testlinien fürs Auge | schwach wahrnehmbar |
| Gruppe 4 = Testlinien fürs Auge | deutlich wahrnehmbar |
| Gruppe 5 = Testlinien fürs Auge | sehr deutlich wahrnehmbar |

Beurteilung / Klassifizierung erfolgte durch drei Personen. Diese beurteilten und klassifizierten die Mess-Ergebnisse unabhängig voneinander!
- Konzentrationsbereich: Unterhalb des Tumor M2PK klinischdiagnostischen Konzentrationsbereiches
- Konzentrationsbereich (Graubereich) im sogenannten Cut-Off Bereich
- Konzentrationsbereich klinisch relevanter Tumor M2PK (Zielbereich)

Die Konzentration der einzelnen Proben wurden zuvor mittels ELSIA technischen Methoden bestimmt. Das heißt, für alle Proben der jeweiligen Gruppe waren die Tumor M2PK Konzentrationen zuvor bestimmt und bekannt.

### Beispiel 7b:

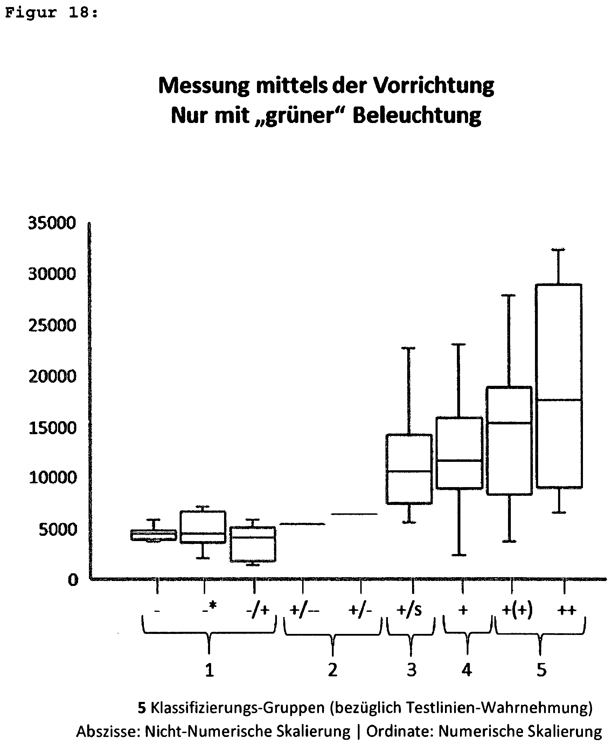

Die Durchführung des Tests erfolgte analog Beispiel 7a, allerdings erfolgte die Auswertung in der erfindungsgemäßen Vorrichtung nur mittels grüner Beleuchtung. Das Ergebnis ist in Figur 18 dargestellt.
5 Klassifizierungs-Gruppen (bezüglich Testlinien-Wahrnehmung)
Abszisse: Nicht-Numerische Skalierung | Ordinate: Numerische Skalierung

| | |
|---|---|
| Gruppe 1 = Testlinien fürs Auge | nicht wahrnehmbar |
| Gruppe 2 = Testlinien fürs Auge | sehr schwach wahrnehmbar |
| Gruppe 3 = Testlinien fürs Auge | schwach wahrnehmbar |
| Gruppe 4 = Testlinien fürs Auge | deutlich wahrnehmbar |
| Gruppe 5 = Testlinien fürs Auge | sehr deutlich wahrnehmbar |

Beurteilung / Klassifizierung erfolgte durch drei Personen. Diese beurteilten und klassifizierten die Mess-Ergebnisse unabhängig voneinander!
- Konzentrationsbereich: Unterhalb des Tumor M2PK klinischdiagnostischen Konzentrationsbereiches
- Konzentrationsbereich (Graubereich) im sogenannten Cut-Off Bereich
- Konzentrationsbereich klinisch relevanter Tumor M2PK (Zielbereich)

Die Konzentration der einzelnen Proben wurden zuvor mittels ELSIA technischen Methoden bestimmt. Das heißt, für alle Proben der jeweiligen Gruppe waren die Tumor M2PK Konzentrationen zuvor bestimmt und bekannt.

**Beurteilung:** Wie aus den Resultaten von Beispiel 7a/7b und insbesondere aus dem Vergleich von Figur 17 und 18 ersichtlich, sind die, durch die Vorrichtung, erzielten Messergebnisse bei der Messung mit dem gesamten Beleuchtungsprotokoll und der zeitlichen Sequenz deulich besser.

Die Messwerte der Messung (nur mit "grüner" Beleuchtung) streuen, wie deutlich sichtbar, wesentlich mehr, als bei der vergleichbaren Messung. Die hohe Streuungs-Varianz führt insgesamt zu einem schlechteren Messergebnis und damit zu einer schlechteren Unterscheidbarkeit (siehe Perzentile der Gruppen) und damit zu einer schlechteren Gesamtbeurteilung der Gruppen.

### Beispiel 8:

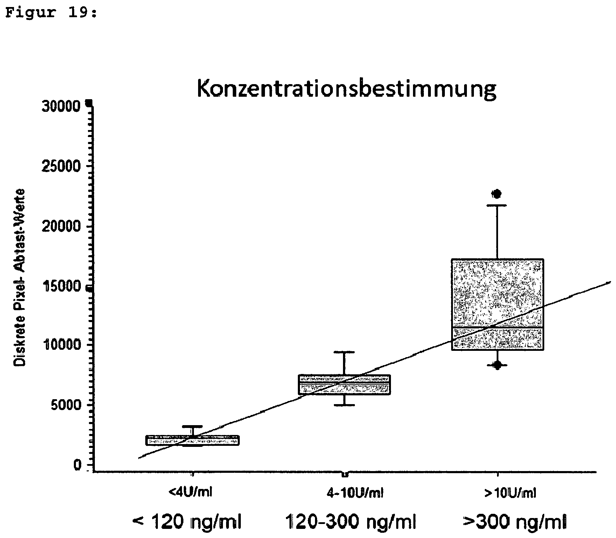

Figur 19 zeigt das Ergebnis Biomarker-Konzentrations-Bestimmung (Tumor - M2PK) in Probanden-Stuhlproben:
Abszisse: Nicht-Numerische Skalierung | Ordinate: Numerische Skalierung
Funktionsgraph: Beschreibt die Abhängigkeit der Konzentration vs. Diskreter Pixel-Abtast-Werte.

Dieser Graph könnte als Eichkurve für die Konzentrations-bestimmung der Tumor M2PK in Proben dienen. Eine Mengenbestimmung mittels der Vorrichtung, der Beleuchtung und der Verwendung eines Bildsensor-Moduls ist möglich.

### Beschreibung der Figuren

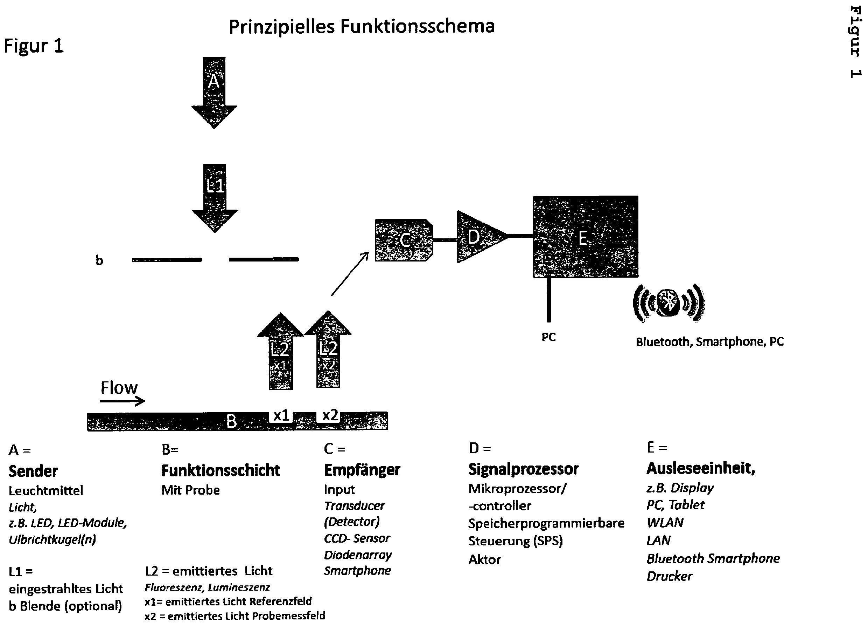

Figur 1 zeigt das prinzipielle Funktionsschema einer Ausführungsform der Erfindung. Der Sender (A) sendet Licht (L1), auf die Funktionsschicht (B), die eine Probe enthält. Der Flüssigkeitsstrom (Flow) ist angezeigt. Die Funktionsschicht enthält zwei Messstellen, nämlich das Probemessfeld (Region of Interest) und ein Referenzfeld. Die Lichtreflektion bzw. -emission wird durch einen Empfänger (C) gemessen und dem Signalprozessor (D) zugeführt. Die Ausgabe erfolgt über eine Ausleseeinheit (E). Diese kann ein Display auf oder an der Vorrichtung sein. Die Vorrichtung kann aber auch gekoppelt sein an einen PC, ein Tablet, ein Smartphone oder ähnliches. Die Verbindung kann dabei kabelgestützt oder kabellos sein.

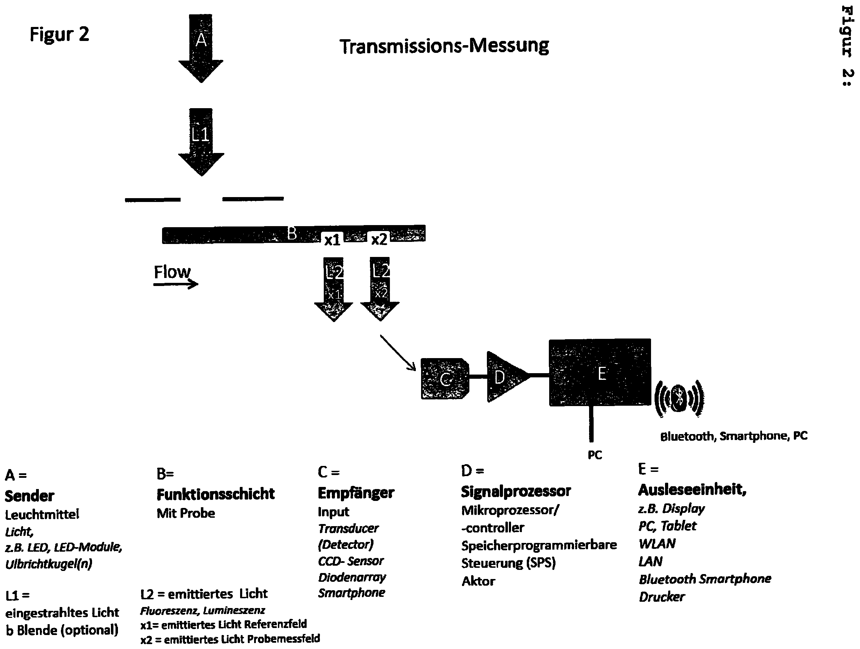

Figur 2 zeigt eine andere Ausführungsform der Erfindung, bei der die Transmission gemessen wird. Der Empfänger (C) befindet sich daher auf der gegenüberliegenden Seite der vollständig oder teilweise transparenten Funktionsschicht.

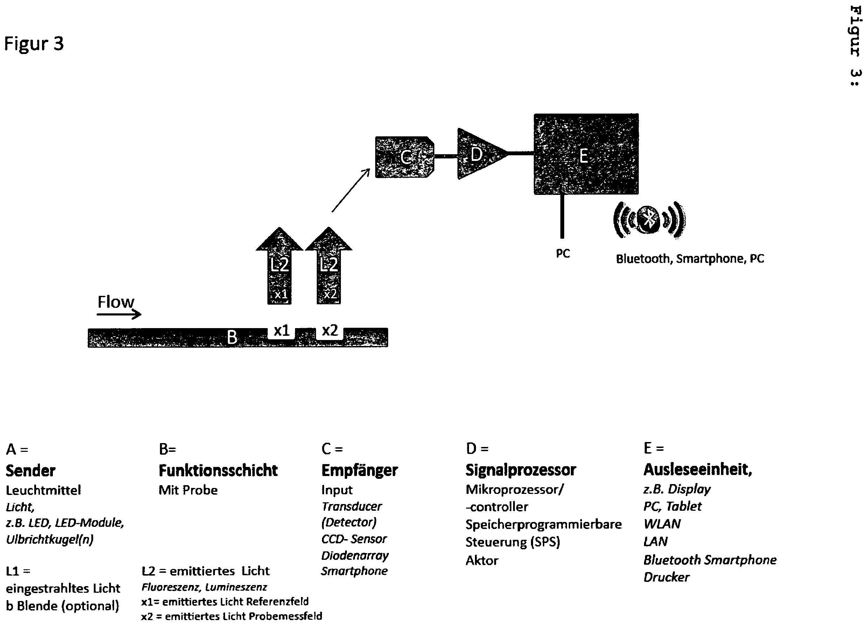

Figur 3 zeigt eine weitere Ausführungsform der Erfindung, bei der eine Lichtemission der Probe stattfindet, beispielsweise durch Biolumineszenz.

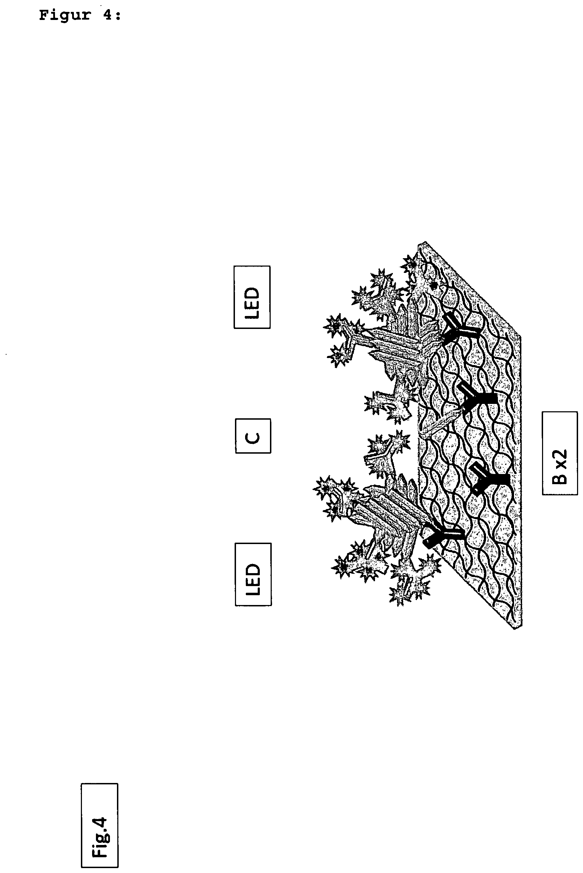

Figur 4 zeigt schematisch die Festphasenfixierung von Antikörpern auf der Funktionsschicht. Unter extremer Vergrößerung wird deutlich, dass die auf der Funktionsschicht typischerweise gebildete "Linie" aus zahlreichen Einzelpunkten besteht.

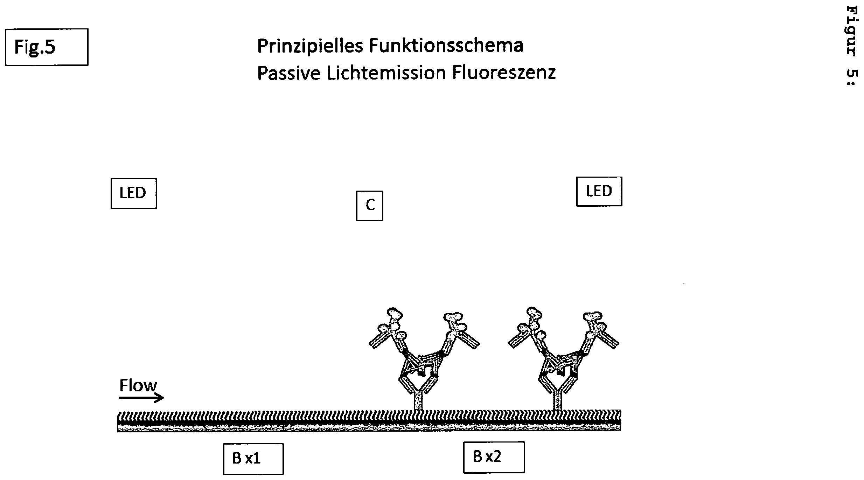

Figur 5 zeigt eine entsprechende Festphasenfixierung von Antikörpern auf der Funktionsschicht in Seitenansicht. In dieser Ausführungsform werden die gebildeten Agglutinate zur Fluoreszenz angeregt.

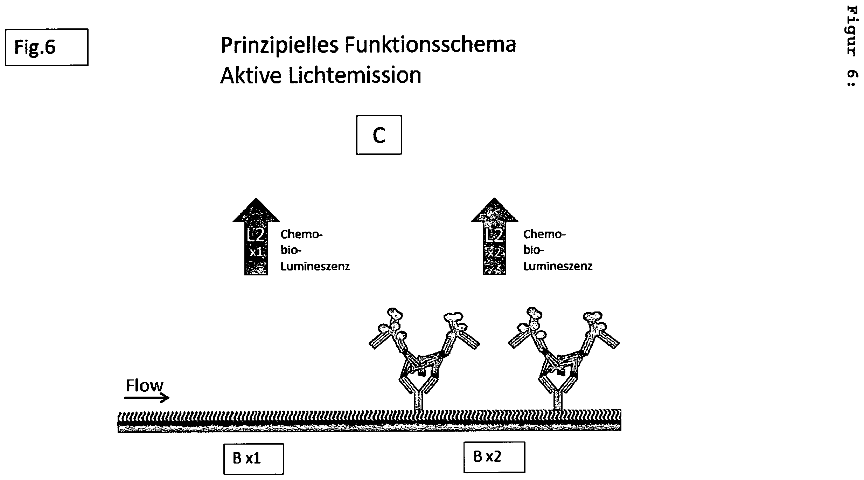

Figur 6 zeigt eine Anordnung analog zu Figur 5, wobei allerdings die gebildeten Agglutinate zur Chemobioluminszenz angeregt werden.

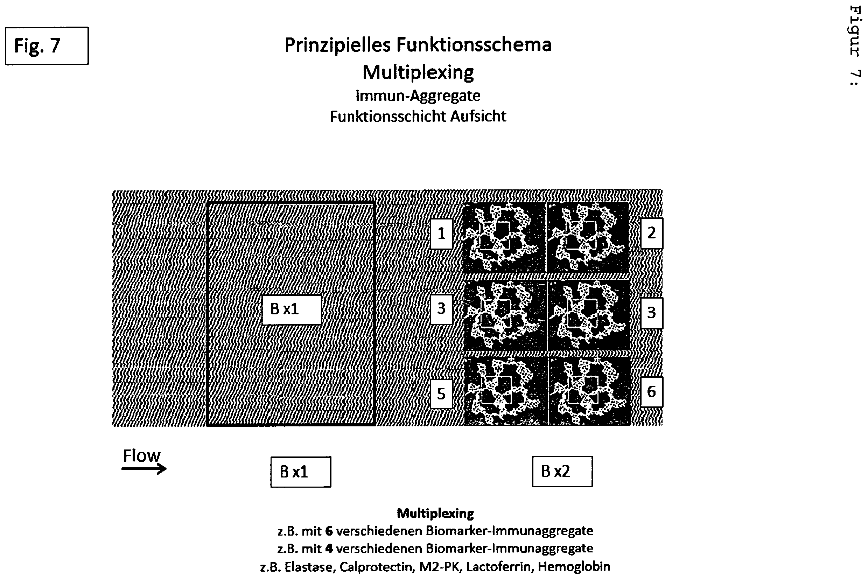

Figur 7 zeigt das Funktionsschema für ein sogenanntes Multiplexing. Hierbei werden verschiedene "Regions of Interest" (ROI) mit verschiedenen Biomarkern versehen. In der Figur 7 werden sechs verschiedene Regionen mit sechs verschiedenen Biomarkern versehen, so dass sechs verschiedene Biomarker gleichzeitig bestimmt werden können.

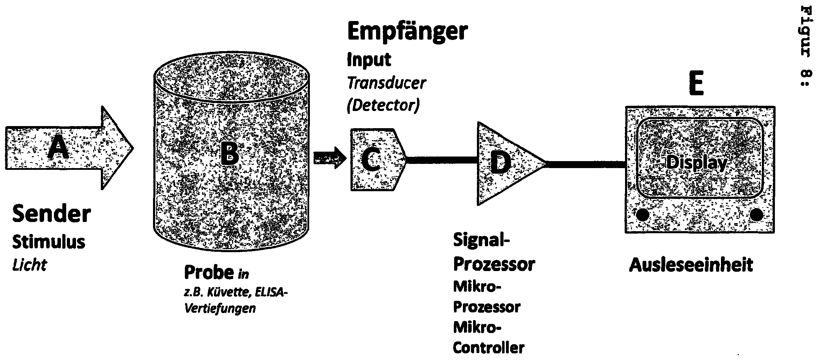

Figur 8 zeigt ein weiteres Funktionsschema einer erfindungsgemäßen Vorrichtung. Hierbei befindet sich die Probe allerdings in einer Küvette, oder in ELISA-wells beispielsweise von einer microwell-Platte. Das Wirkungsprinzip der Erfindung ist allerdings identisch.
Figur 9 zeigt eine andere Ausführungsvariante eines erfindungsgemäßen Analyseautomaten. In dieser Ausführungsform ist die Funktionsschicht senkrecht angeordnet. Die Lichteinstrahlung erfolgt daher in horizontaler Richtung, durch ein ringförmig angeordnetes LED-Modul. Im Zentrum des Ringes ist das Empfängermodul angeordnet. Die weitere Bildverarbeitung erfolgt analog zu den oben bereits beschriebenen Systemen.
Figur 10 zeigt eine weitere beispielshafte Ausführungsform. Im Unterschied zu der in Figur 9 dargestellten Variante ist die Funktionsschicht aus Trägerpartikels und/oder Beads beladen, welche aus der Chromatografie bekannt sind. Auf diese Weise wird eine verbesserte Auftrennung der Probe bewirkt. Die Messung und Auswertung erfolgt analog zu den oben beschriebenen Ausführungsformen.

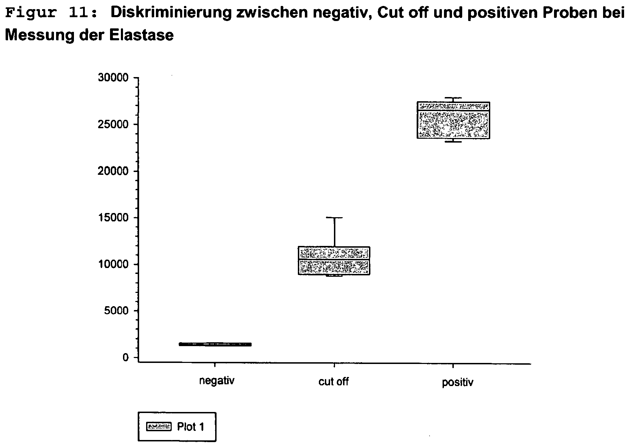

Figur 11 zeigt Messwerte bei Bestimmung der Elastase mittels der erfindungsgemäßen Vorrichtung. Deutlich wird der signifikante Unterschied der Messergebnisse eines positiven Befundes gegenüber einem negativen Befund.

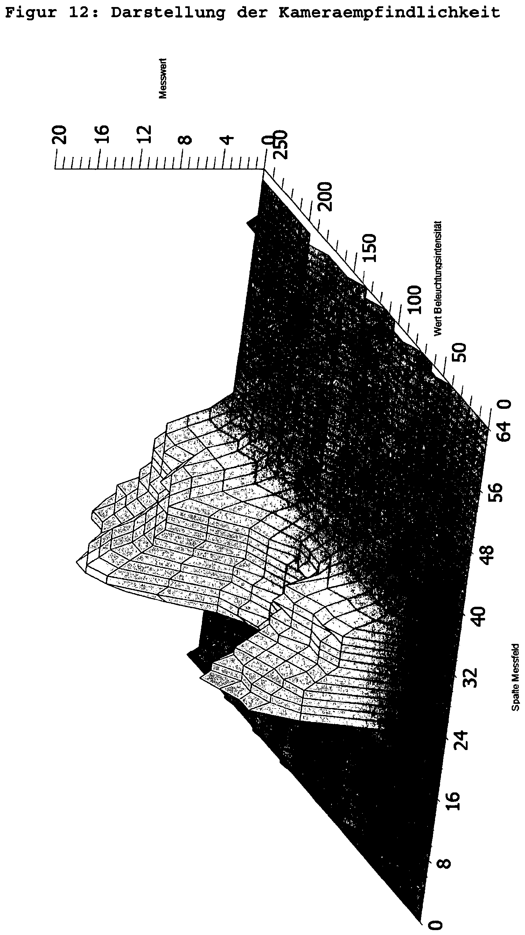

Figur 12 zeigt die Lichtempfindlichkeit eines beispielhaften Sensormodules.

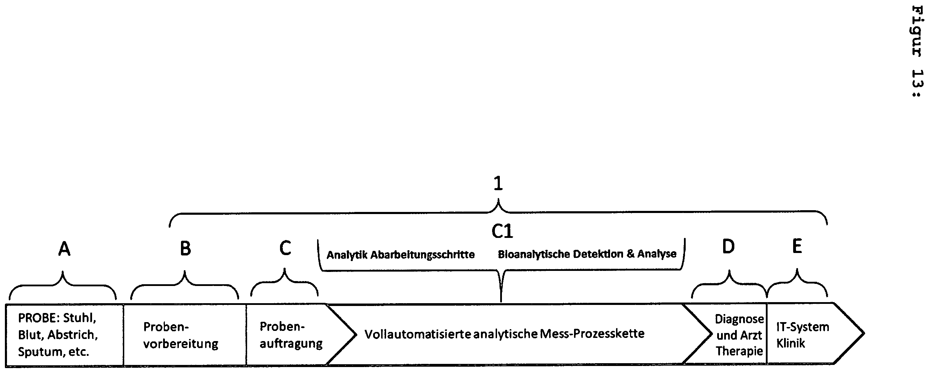

Figur 13 zeigt einen automatisierten Workflow unter Verwendung der erfindungsgemäßen Vorrichtung.

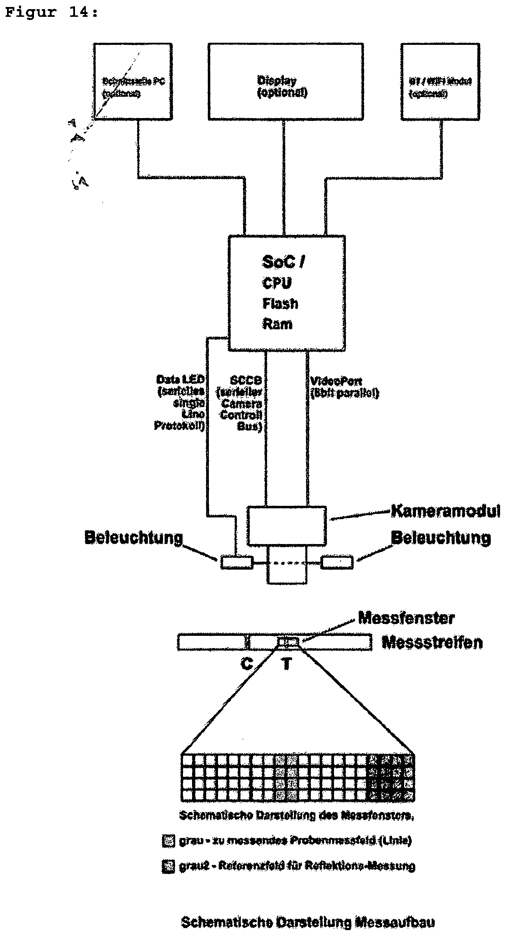

Figur 14 zeigt den Messaufbau einer erfindungsgemäßen Vorrichtung ohne das Gehäuse.

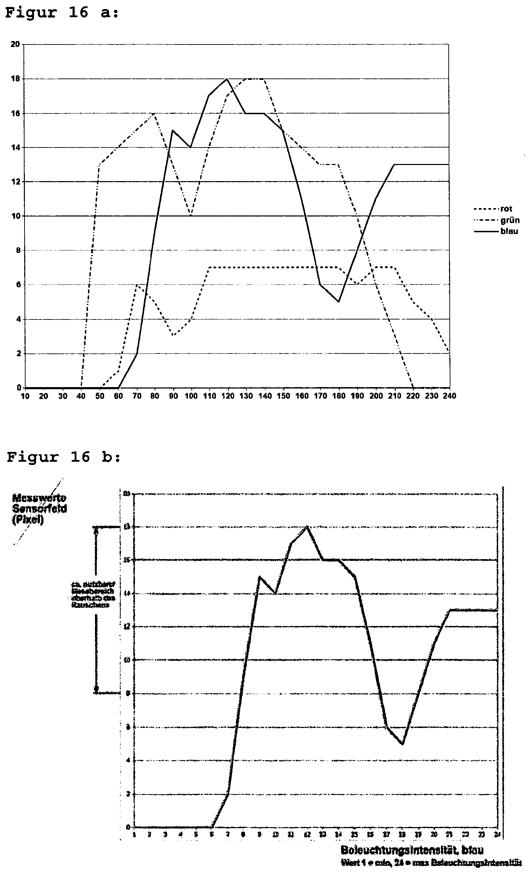

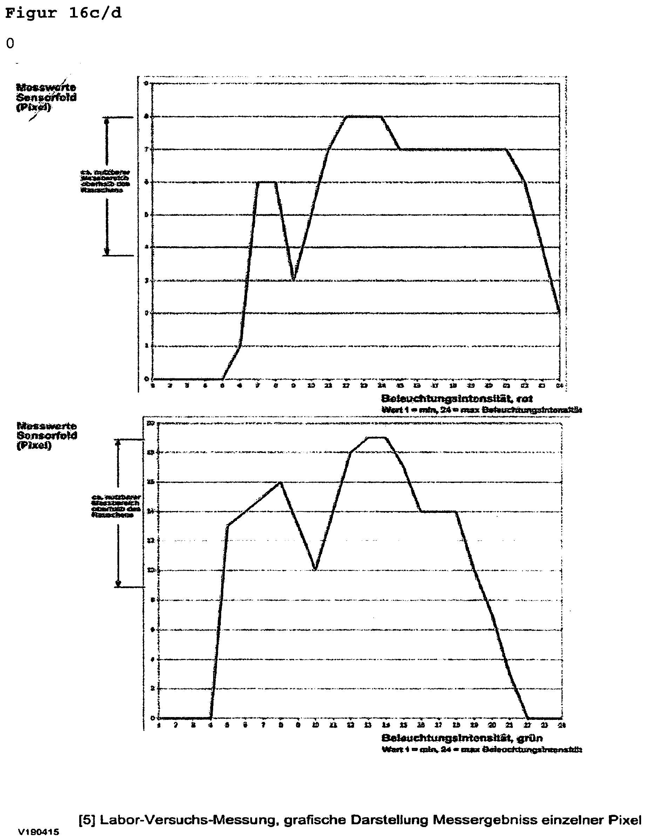

Figuren 15 und 16 (a, b, c, d) zeigen Messungen einzelner Pixel von CMOS-Bildsensoren in Abhängigkeit von der Beleuchtung zum Auffinden der optimalen Beleuchtungsstärke bei unterschiedlichen Farben zur Minimierung des Rauschens.
   Auf der x Achse wird die relative Beleuchtungsintensität für rotes, blaues, grünes Licht gezeigt. Die y Achse zeigt das nutzbare Messsignal in Form von Pixel-Abtastwerten (oberhalb des elektronischen Rauschens) innerhalb einer "Region of Interest" ROI. Auswertung erfolgt mittels des Schwellenwertverfahrens der digitalen Bildverarbeitung (https://de.wikipedia.org/wiki/Schwellenwertverfahren) .
Figuren 17 und 18 zeigen Messergebnisse gemäß Beispiele 7.
Figur 19 zeigt die Konzentrationsbestimmung eines Analyten in der Probe mittels der erfindungsgemäßen Vorrichtung, gemäß Beispiel 8.

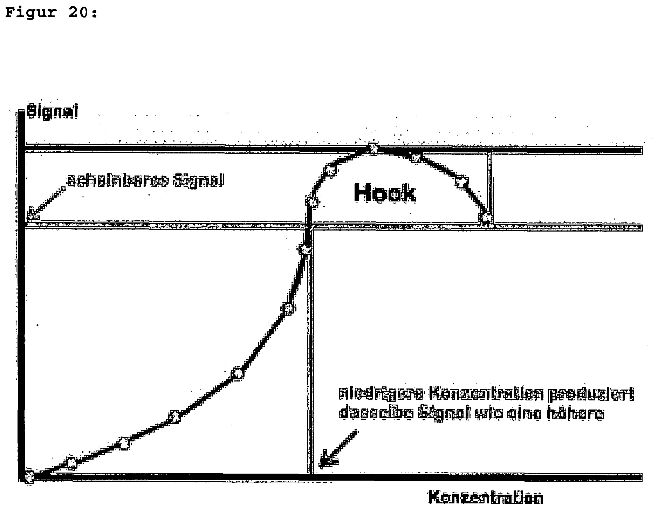

Figur 20 zeigt eine häufige Fehlerquelle bei immunologischen Assays, nämlich den sogenannten Hook-Effekt. Immunologische Assays z.B. lateralflow Assays (LFA) können unter Umständen ein falsch-negatives Ergebnis liefern. Neben anderen Ursachen, wie Kreuz-Reaktivität oder Antikörper-Interferenz ist für dieses Phänomen der sogenannte High-Dose Hook Effekt verantwortlich. Hierbei zeigt der Test aufgrund von Übersättigung der Antikörper mit der in zu hoher Konzentration vorhandenen Antigen ein falsch-negatives Ergebnis. (Lit. z.B. Christian Silberbauer et al, Wehrmedizinische Monatsschrift 12/2018)
   Bei visueller Auslesung / Auswertung ist neben der individuellen Bewertung die obere Nachweisgrenze (upper detection limit, UDL) und die untere Nachweisgrenze (Lower Detection Limit, LDH) und der Hook-Effekt von Bedeutung.
   Ein bisheriges großes Problem ist die visuelle Beurteilung und Klassifizierung, da dies bei Inaugenscheinnahme durch individuelle Wahrnehmung der Messergebnisse zustande kommt, aufgrund der Bestimmung des Lateral-Flow.

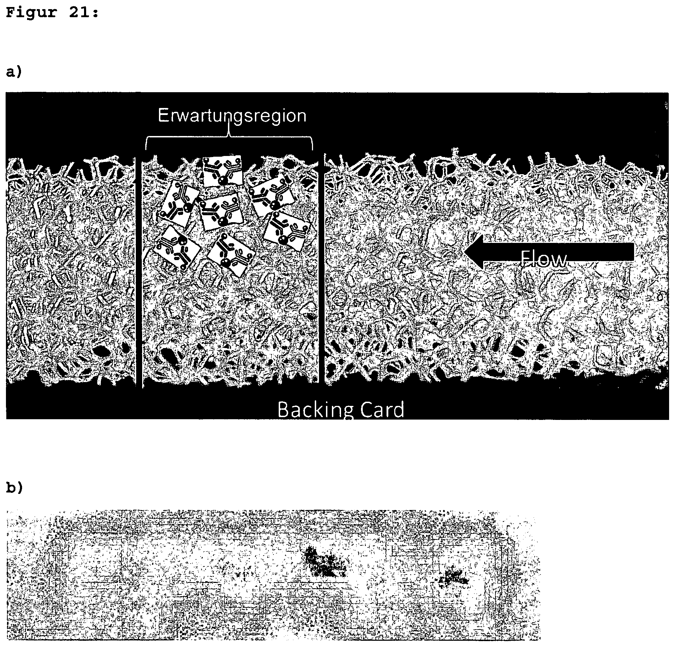

Figur 21a zeigt schematisch die "Testlinie", die durch Auftragung einer Antikörperlösung (mittels Präzision-Nanodispensierung) auf die Mikro-Zellulose Membran aufgebracht wird.
   Dieses führt zur Fixierung der Fängermoleküle (Antikörper) in die 3D Struktur der offenporigen Zellulose-Membran; nach Auftragung der Probe auf das Sample-Pad der Membran.
   Diese Fängermoleküle (Antikörper) binden das mit kollidialen Gold und einem zweiten Fängermolekül (zweiter Antikörper) markierten, zu bestimmenden Bio-Analyten.
   Gemäß der nach Herrn Heidelberger benannten "Heidelberger Kurve" entsteht ein analyt-konzentrationsabhängiges Immunaggregat (Präzipitat) in der 3D Struktur der offenporigen Lateral-Flow Mikrozellulose Membran. Dieses Aggregat bestehend aus dem Bio-analyten, dem Gold und dem zweiten Antikörper.
   Das sich zeitabhängig, bildende Immunaggregat besitzt, bestimmte charakteristische, optische, physikalische Eigenschaften. Die Lichtabsorptionseigenschaften werden besonders deutlich bei Beleuchtung durch Licht, denn es gibt Unterschiede zwischen den Absorptions- / Reflektion-Eigenschaften, der in der Erwartungsregion befindlichen Immunaggregate, und der Region, der mikrozellulose Membran, in der kein Gold-ImmunAggregat anwesend ist. Die flächenbezogenen Unterschiede in den optisch, physikalischen Eigenschaften lassen sich gern, der erfinderischen Messanordnung und -verfahren digital optisch bestimmen.
   Wie die mikroskopische Aufnahme in Figur 21b zeigt, ist die zu erwartende "Ergebnis-Testlinie", bedingt durch die Präzisionsnano-dispensierung-Herstellungsverfahren (durch den Herstellungsprozess bedingt), eigentlich keine Linie sondern besteht aus Punktwolken. Dieses führt neben, zusätzlich zu den Randeffekten dieser Punktwolken, zu Ungenauigkeiten bei der visuellen, individuellen Empfindung und Wahrnehmung der Testlinien bei Inaugenscheinnahme.
   Da die "Testlinien", wie die mikroskopische Abbildung zeigt, keine Linie darstellt, sondern aus einer Aneinanderreihung von "Punkten" bzw. "Punktwolken", wurden erfindungsgemäß besondere Vorgehensweisen (durch vielfältige Testreihen bestimmt). Dies erfolgt beim Abtastungsschritt / -samplingprozess im Bildsensor-Modul und im Prozessor zur Verbesserung der Messaufgaben.
   Bezüglich dieses Mangels und weiterer Mängel wurde der erfinderische Messaufbau und Algorithmen, die zu besseren Ergebnissen führen, verwendet.

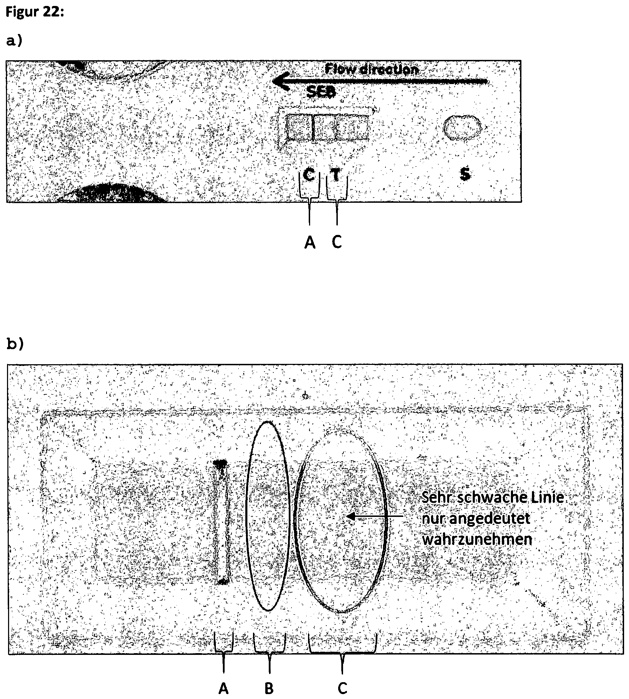

Figur 22a zeigt die Abbildung einer Test-Kassette mit dem eindeutigen Testergebnis
   Visuelle, individuelle Wahrnehmung des Testergebnisses:
   A: Kontrolllinienregion, bei sichtbarer Linie zeigt dieses an, dass die Funktionalität des Tests gewährleistet ist
   C: Erwartungsregion (Region of Interest [ROI]), bei der das Auftreten einer Linie erwartet wird oder nicht erwartet wird, nach einem bestimmten Zeitraum nach Auftragen der flüssigen Probe
Figur 22b zeigt die vergrößerte Abbildung eines Testfeldes einer Test-Kassette
   Nicht eindeutiges Testergebnis eines Testergebnisses
   Testlinie (Immun-Aggregation) nur angedeutet zu erkennen / wahrzunehmen
   Dieses Ergebnis der Inaugenscheinnahme erfolgt sehr häufig bei Proben, derer Biomarker-Konzentrationen klinisch sehr nahe am sogenannten "Cut-Off" liegen.
      A: Kontrolllinie: wird erfindungsgemäß normaler Weise nicht zur Messung genutzt
      B: Referenzmessfeld-Region: wird erfindungsgemäß zur digitalen Messung genutzt (Region of Interest [ROI])
      C: Erwartungsmessfeld-Region (ROI): wird erfindungsgemäß zur digitalen Messung genutzt
   Erfindungsgemäß werden B und C digital verglichen (Digital berechnet (Bool'sche-Algebra) und mittels Logik-Bausteinen weiterverarbeitet). Mittels dieser Schritte ("Rechenschritte"), erhält man numerische Werte nach digital-analoger Wandlung.
   Die Nummerischen Werte werden erfindungsgemäß,
      1) durch eine erfindungsgemäße digitalgesteuerte Vorrichtung und
      2) durch die Verwendung eines Bildsensor-Modules (siehe Abbildung zum Bildsensor-Modul) erhalten.

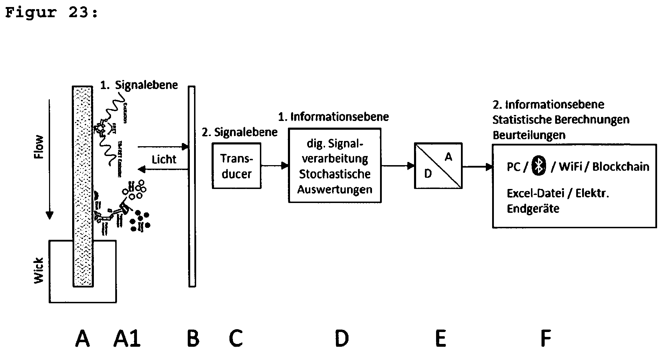

Figur 23 zeigt eine beispielhafte Ausführungsvariante eines Analysevollautomaten
   Flüssige Probe / Reagenzien u.s.w. / Mess-Prozess-Kette zum Ziel: Analyse der quantitativen-qualitativen Bestimmung. (Es versteht sich von selbst, dass jeweils nur ein Reportersystem verwendet wird und nicht wie dargestellt zwei verschiedene Reportersysteme (da Funktionsskizze)), z.B. durch PC-gesteuerte Mikropumpe
   A: Funktionsschicht, z.B. offenporiges Glass. In der 3-D-Schicht wurde, z.B. mittels Silan-Chemie Fängermoleküle orts-/flächengebunden kovalent fixiert
   A1: Fängermoleküle mit Reportersystemen FRET (Förster Resonance Transfer) Fluorence Engergy Transfer, TR-FRET Emission Chemilumineszenz, Fluoreszenz / ECL, spezielle Western Blotting- Methoden und unterschiedliche Substrate, die zu wasserunlöslichen Reaktionsprodukten führen
   B: Sender, Beleuchtungsvorrichtung, RGB-LED-Modul (Digital gesteuerte Treiber)
   C: Empfänger, Detektor, z.B. Avalance Photo Multiplier Tube (APDs), bevorzugt Bildsensor Modul (linsenbasierte Kamera, maskenbasierte Kamera) Optical Sensors, Multi-Pixel Photon Counters (MPPCs/SiPMs), Infrared Detector, sCMOS Camera, CCD Camera, Si Photodiode, TDI-CCD and CMOS readout circuits, usw. Drartige Empfängermodule sind ausführlich im Stand der Technik beschrieben.
   D: Prozessor, digitale Verrechnung, kleinflächig bezogene Informationen (aufgrund der Pixel-Array Anordnung) werden gefiltert, mittels stochastischer Methoden analysiert, und digital verstärkt.
   digitale Verrechnung, großflächig bezogene Informationen (aufgrund der Referenzflächen ./. Erwartungsregion Messfläche) werden gefiltert, mittels stochastischer Methoden analysiert, und digital verstärkt.
   Bool'sche Algebra, logische Bausteine, Verknüpfungen, Kalibrierung mittels Kalibrator, Verrechnung mittels Eichkurve, usw.
   E: Digital-Analog-Wandler
   F: PC / Schnittstellen / weitere Verarbeitung mittels Algorithmen, im Besonderen Blockchain-Technologie (Zusammenführung von Patienten-Messdaten und daraus folgenden Diagnosen können global verglichen, gesichert, historisiert und archiviert werden, genutzt als Golden Source) / Nummerischer Messwert / auch künstliche Intelligenz-Algorithmen (neuronale Netze, Mustererkennung, Algorithmen, usw.)

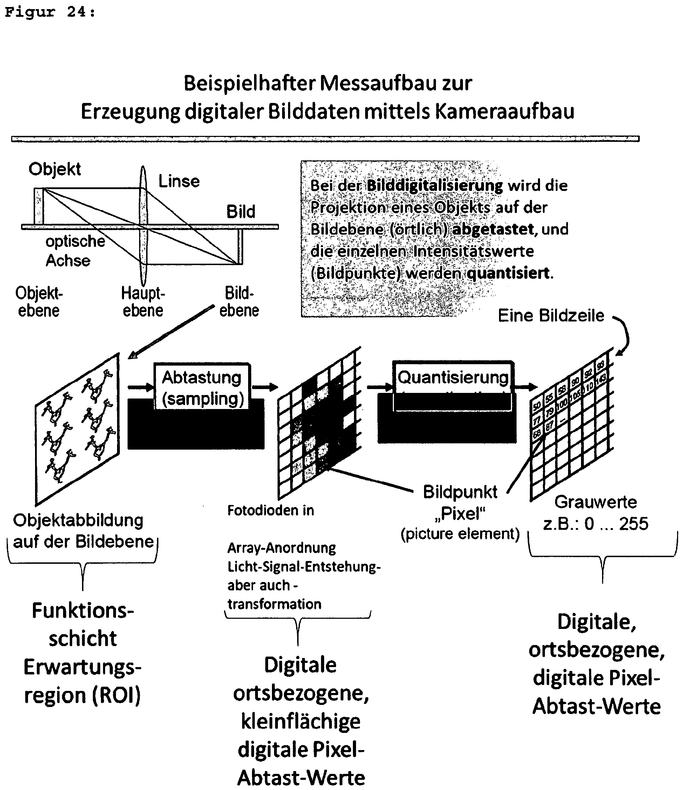

Figur 24 zeigt einen beispielhaften Messaufbau zur Erzeugung digitaler Bilddaten mittels Kameraaufbau

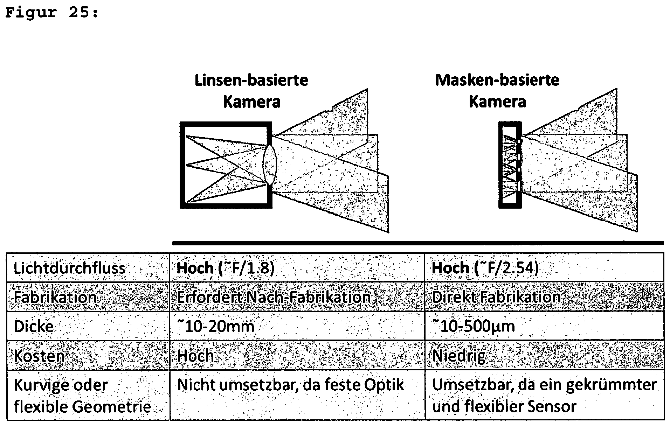

Figur 25 zeigt Ausführungsvarianten des Bildsensor-Moduls zur Erzeugung digitaler Bilddaten mittels unterschiedlicher Vorrichtungsvarianten.

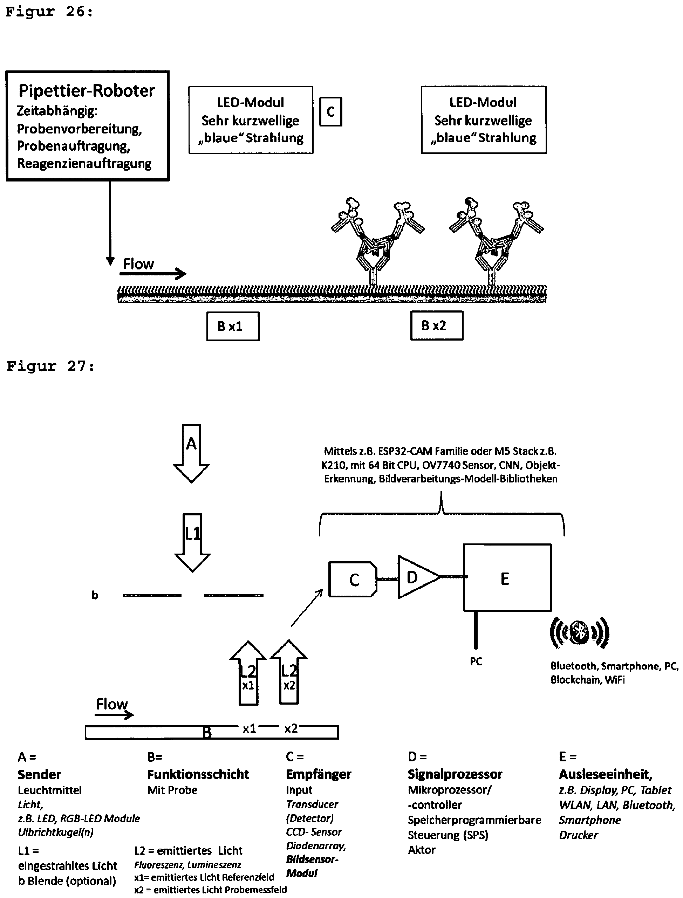

Figur 26 zeigt das prinzipielle Funktionsschema mittels passiver Lichtemission, z.B. Fluoreszenzmessung. Beleuchtung / Anregung erfolgt mittels LED-Modul oder einer im UV-Bereich emittierenden Leuchtdiode. Die verbesserte Anregung eines ausgewählten Chromophors im ausgewählten Reportersystem (durch Testreihen ermittelt)
Figur 27 zeigt das prinzipielles Funktionsschema der Reflexions-/ Absorptionsmessung mittels der erfindungsgemäßen Vorrichtung.

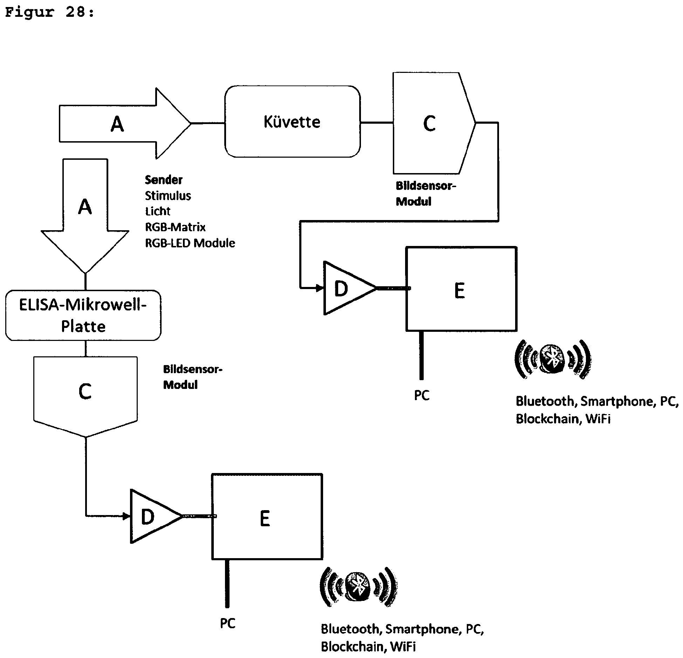

Figur 28 zeigt das prinzipielle Funktionsschema der qualitativen und quantitativen Absorptionsmessung. Eine besondere Funktionsvariante: Verwendung von modernen, Liquid-handling Technologien z.B. für hoch-durchsatz-Technologien. Bei diesen werden z.B. Flüssigkeits-Alliquote aus z.B. Mikrowell-Platten mittels Beschallung dieser, auf eine darüber liegende Schicht, ortsbezogen transferiert/überführt. Diese so vorbereitete Schicht (an der z.B. ortsbezogen Stoffe haften) kann als Funktionsschicht im beschriebenen Messaufbau verwendet werden, siehe Figur 28. Somit lässt sich die beschriebene Messvorrichtung auch bei in hoch-durchsatz-Prozesstechniken z.B. beim Screenen von Flüssigkeits-Überständen von Hybridoma-Zellen verwenden. Diese Hybridoma-Zellen wachsen in z.B. Mikrowell-Platten.
   Zwei Möglichkeiten: Entweder vertikale Beleuchtung Mikrowell-Platte und/oder horizontale Beleuchtung von Küvette/-n.

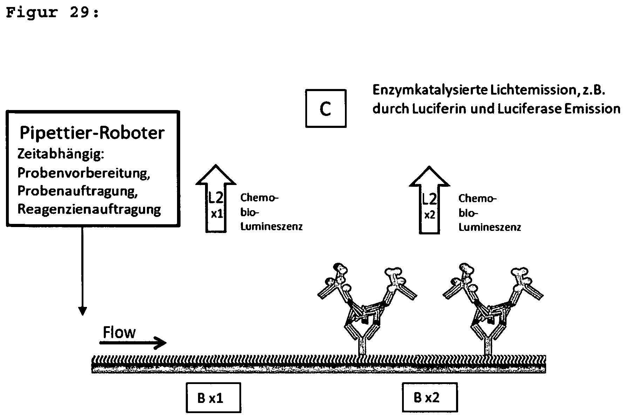

Figur 29 zeigt das prinzipielle Funktionsschema der aktiven Lichtemission, z.B. durch Chemo-Lumineszenz und Bio-Lumineszenz bei Abarbeitung der vollautomatischen diagnostischen Prozesskette.

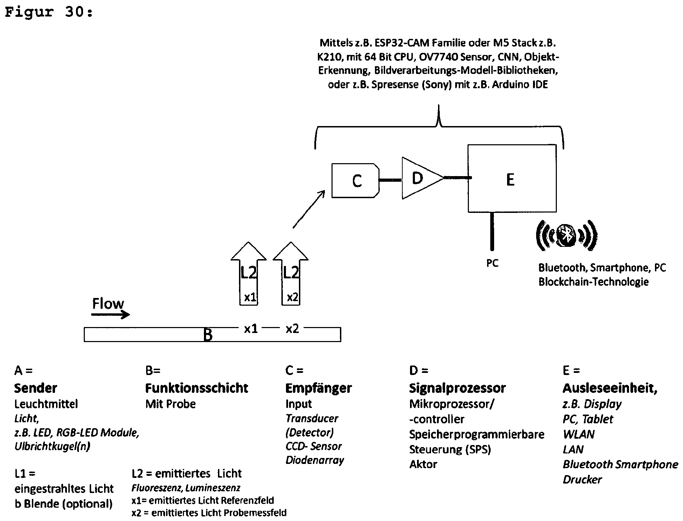

Figur 30 zeigt das prinzipielle Funktionsschema der Lichtmessung mittels der erfindungsgemäßen Vorrichtung. Messergebnisse können kabellos auf mobile Geräte übertragen werden. Mittels Blockchain-Technologie kann eine fälschungssichere Speicherung erfolgen.

### Stand der Technik

Zusätzlicher relevanter Stand der Technik ist beschrieben in den folgenden Druckschriften:
WO2016/205736A1,
DE102019005761A1, DE102018000815A1, DE102016015060A1,
WO2016015701A1, US20150219658A1, WO2012022285A2,
US20080153119A1, US20080113389A1, EP1474235A1, DE2032160U1,
US20200107760A1, US20200156074A1, US20200174005A1,
US2010/267049, WO2018/150787, EP2587249,
Sensors, 2011, 11, 6396-6410.

## Patentansprüche

1. Vorrichtung zur Auslesung eines Test-Kits zum Nachweis von Biomarkern, enthaltend
ein lichtundurchlässiges Gehäuse,
eine Haltevorrichtung zum Fixieren einer Funktionsschicht mit oder ohne Kassette,
eine Funktionsschicht mit darauf fixierten Antikörpern zum immunologischen Nachweis eines Antigens und/oder eine eine Funktionsschicht mit darauf fixierten Antigenen zum immunologischen Nachweis eines Antikörpers,
einen Sender zur gesteuerten Beleuchtung der Funktionsschicht, wobei der Sender gesteuert Licht unterschiedlicher Wellenlänge aussenden kann,
einen Empfänger zur Bestimmung des reflektierten oder transmittierten Lichtes,
einen Prozessor zur Steuerung von Sender, Empfänger und zur Signalaufbereitung,
eine Anzeigevorrichtung zur Anzeige des Ergebnisses,
wobei die Vorrichtung mittels einer auf dem Prozessor ablaufenden Software gesteuert wird,
wobei die Steuerung
den Sender nacheinander Licht von mindestens 2 verschiedenen Wellenlängenbereichen aussenden lässt,
die Signalaufbereitung nur das Helligkeitsignal (Y-Signal) des Empfängers auswertet,
den Messzyklus ein- oder mehrfach (2 - 5-fach) wiederholt,
die gemessenen Signale per digitaler Bildverarbeitung auswertet und anzeigt.

2. Vorrichtung gemäß Anspruch 1, wobei als Sender RGB-LED Module verwendet werden.

3. Vorrichtung gemäß Anspruch 1 oder 2, wobei als Funktionsschicht eine Nitrozellulose Membran verwendet wird, auf der sich festphasengebundene Antikörper sowie mobile antikörpergebundene Goldpartikel oder auf der sich festphasengebundene Antigene sowie mobile antikörpergebundene Goldpartikel befinden.

4. Vorrichtung gemäß Anspruch 1 oder 2, wobei die Funktionsschicht mehrere festphasengebundene Antikörper und/oder Antigene an unterschiedlichen Positionen aufweist.

5. Vorrichtung gemäß Anspruch 4, wobei die Funktionsschicht mehrere festphasengebundene Antikörper und/oder Antigene an unterschiedlichen Positionen aufweist, wobei jede Position als Linie ausgebildet ist und wobei die Linien in Laufrichtung hintereinander (leiterförmig) angeordnet sind und wobei die einzelnen Linien unterschiedliche Konzentrationen an Antikörper und/oder Antigene aufweisen.

6. Vorrichtung gemäß Anspruch 1 bis 5, wobei als Empfänger ein CMOS oder ein sCMOS-Chip verwendet wird von dessen Signalen nur diejenigen verwendet werden, die dem Messfeld zugeordnet sind.

7. Vorrichtung gemäß einem der Ansprüche 1 bis 6, wobei zur aller Komponenten und zur Auswertung der Messungen eine integrierte Entwicklungsumgebung verwendet wird.

8. Vorrichtung gemäß einem der Ansprüche 1 bis 7, wobei das lichtundurchlässige Gehäuse Halteelemente zur Fixierung der Funktionsschicht aufweist.

9. Verwendung einer Vorrichtung gemäß einem der Ansprüche 1 bis 8 zum Nachweis von Hämoglobin, M2-PK, Calprotectin, Lactoferrin, Helicobacter pylori, Adenoviren, Clostidin dificile, Norovirus, EHEC, Coronaviren oder zu Durchführung von umweltanalytischen, forensischen oder lebensmittelanalytischen Schnelltests, Schwangerschafts-, Doping- oder Drogenschnelltests.

10. Verwendung einer Vorrichtung gemäß einem der Ansprüche 1 bis 8 zum synchronen Nachweis von mindestens 2 Biomarkern, ausgewählt aus der Gruppe "Hämoglobin, M2-PK, Calprotectin, Lactoferrin, Helicobacter pylori, Adenoviren, Clostidin dificile, Norovirus, EHEC, Coronaviren" oder zur Durchführung von umweltanalytischen, forensischen oder lebensmittelanalytischen Schnelltests, Schwangerschafts-, Doping- oder Drogenschnelltests, wobei mindestens zwei verschiedene Biomarker bestimmt werden.

## Claims

1. A device for reading out a test kit for the detection of biomarkers, containing
an opaque housing,
a holding device for fixing a functional layer with or without a cassette,
a functional layer with antibodies fixed thereon for the immunological detection of an antigen and/or a functional layer with antigens fixed thereon for the immunological detection of an antibody,
a transmitter for controlled illumination of the functional layer, wherein the transmitter can emit light of different wavelengths in a controlled manner,
a receiver to determine the reflected or transmitted light,
a processor for controlling the transmitter, the receiver, and for signal conditioning,
a display device for displaying the result,
wherein the device is controlled by means of software running on the processor,
wherein the control causes the transmitter to successively emit light of at least 2 different wavelength ranges,
the signal conditioning only evaluates the brightness signal (Y-signal} of the receiver,
repeats the measuring cycle once or several times (2 - 5 times),
evaluates and displays the measured signals via digital image processing.

2. The device according to claim 1, wherein RGB LED modules are used as transmitters.

3. The device according to claim 1 or 2, wherein as a functional layer, a nitrocellulose membrane is used on which solid phase-bound antibodies as well as mobile antibody-bound gold particles or on which solid phase-bound antigens as well as mobile antibody-bound gold particles are located.

4. The device according to claim 1 or 2, wherein the functional layer comprises a plurality of solid phase-bound antibodies and/or antigens at different positions.

5. The device according to claim 4, wherein the functional layer comprises several solid phase-bound antibodies and/or antigens at different positions, each position being formed as a line and the lines being arranged one behind the other (ladder-shaped) in the running direction, and the individual lines having different concentrations of antibodies and/or antigens.

6. The device according to claim 1 to 5, wherein as the receiver, a CMOS or an sCMOS chip is used, of the signals of which only those are used which are assigned to the measuring field.

7. The device according to any one of claims 1 to 6, wherein an integrated development environment is used for all components and for evaluating the measurements.

8. The device according to any one of claims 1 to 7, wherein the opaque housing comprises retaining elements for fixing the functional layer.

9. Use of a device according to any one of claims 1 to 8 for the detection of hemoglobin, M2-PK, calprotectin, lactoferrin, Helicobacter pylori, adenoviruses, Clostridioides difficile, norovirus, EHEC, coronaviruses or for the performance of rapid environmental, forensic or food analytical tests, pregnancy, doping or drug rapid tests.

10. Use of a device according to any one of claims 1 to 8 for the synchronous detection of at least 2 biomarkers selected from the group "hemoglobin, M2-PK, calprotectin, lactoferrin, Helicobacter pylori, adenoviruses, Clostridioides difficile, norovirus, EHEC, coronaviruses" or for the performance of environmental analytical, forensic, or food analytical rapid tests, pregnancy, doping or drug rapid tests, wherein at least two different biomarkers are determined.

## Revendications

1. Dispositif de lecture d'un kit de test pour la détection de biomarqueurs, contenant
un boîtier opaque,
un dispositif de maintien pour fixer une couche fonctionnelle avec ou sans cassette,
une couche fonctionnelle sur laquelle sont fixés des anticorps pour la détection immunologique d'un antigène et/ou une couche fonctionnelle sur laquelle sont fixés des antigènes pour la détection immunologique d'un anticorps,
un émetteur pour l'illumination commandée de la couche fonctionnelle, l'émetteur pouvant émettre de la lumière de différentes longueurs d'onde de manière commandée,
un récepteur pour la détermination de la lumière réfléchie ou transmise,
un processeur pour la commande de l'émetteur, du récepteur et pour le traitement du signal,
un dispositif d'affichage pour l'indication du résultat,
dans lequel le dispositif est commandé au moyen d'un logiciel fonctionnant sur le processeur, la commande amenant l'émetteur à émettre successivement de la lumière d'au moins deux gammes de longueurs d'onde différentes,
le traitement du signal n'évalue que le signal de luminosité (signal Y) du récepteur,
répète le cycle de mesure une ou plusieurs fois (2 à 5 fois),
évalue et affiche les signaux mesurés par traitement d'image numérique.

2. Dispositif selon la revendication 1, dans lequel des modules LED de type RVB sont utilisés comme émetteurs.

3. Dispositif selon la revendication 1 ou 2, dans lequel comme couche fonctionnelle est utilisée une membrane de nitrocellulose sur laquelle se trouvent des anticorps liés à une phase solide ainsi que des particules d'or mobiles liées à des anticorps ou sur laquelle se trouvent des antigènes liés à une phase solide ainsi que des particules d'or mobiles liées à des anticorps.

4. Dispositif selon la revendication 1 ou 2, dans lequel la couche fonctionnelle comprend plusieurs anticorps et/ou antigènes liés à la phase solide à des positions différentes.

5. Dispositif selon la revendication 4, dans lequel la couche fonctionnelle comprend plusieurs anticorps et/ou antigènes liés à la phase solide à des positions différentes, dans lequel chaque position est réalisée sous forme de ligne et dans lequel les lignes sont disposées les unes derrière les autres (en échelle) dans le sens de la marche et dans lequel les lignes individuelles ont des concentrations différentes d'anticorps et/ou d'antigènes.

6. Dispositif selon une des revendications 1 à 5, dans lequel comme récepteur est utilisée une puce CMOS ou sCMOS dont seuls les signaux qui sont associés au champ de mesure sont utilisés.

7. Dispositif selon une des revendications 1 à 6, dans lequel un environnement de développement intégré est utilisé pour tous les composants et pour l'évaluation des mesures.

8. Dispositif selon une des revendications 1 à 7, dans lequel le boîtier opaque comprend des éléments de maintien pour fixer la couche fonctionnelle.

9. Utilisation d'un dispositif selon une des revendications 1 à 8 pour la détection d'hémoglobine, de M2-PK, de calprotectine, de lactoferrine, d'Helicobacter pylori, d'adénovirus, de Clostridioides difficile, de norovirus, d'EHEC, de coronavirus ou pour la réalisation de tests rapides d'analyse environnementale, médico-légale ou alimentaire, de tests rapides de grossesse, de dopage ou de dépistage de drogues.

10. Utilisation d'un dispositif selon une des revendications 1 à 8 pour la détection synchrone d'au moins 2 biomarqueurs choisis dans le groupe «hémoglobine, M2-PK, calprotectine, lactoferrine, Helicobacter pylori, adénovirus, Clostridioides difficile, Norovirus, EHEC, Coronavirus» ou pour la réalisation de tests rapides d'analyse environnementale, médico-légale ou alimentaire, de tests rapides de grossesse, de dopage ou de dépistage de drogues, au moins deux biomarqueurs différents étant déterminés.
